# EUROPEAN PATENT SPECIFICATION

(11) **EP 2 739 733 B1**
(45) Date of publication and mention of the grant of the patent: **22.11.2017**
(21) Application number: 12819442.0
(22) Date of filing: 03.08.2012
(51) Int. Cl.: C12N 15/11, C12N 15/12, C12N 5/10, C12P 21/00

(54) **NUCLEIC ACID COMPOSITIONS, METHODS AND KITS FOR RAPID PAIRING OF AFFINITY AGENTS**
NUKLEINSÄUREVERBINDUNGEN, VERFAHREN UND KITS ZUR SCHNELLEN PAARUNG VON AFFINITÄTSWIRKSTOFFEN
COMPOSITIONS D'ACIDE NUCLÉIQUE, PROCÉDÉS ET KITS POUR L'APPARIEMENT RAPIDE D'AGENTS D'AFFINITÉ

(30) Priority: 03.08.2011 US 201161514717 P
(43) Date of publication of application: 11.06.2014
(73) Proprietor: Texas Biomedical Research Institute, San Antonio, Texas 78227-5301 (US)
(72) Inventor: HAYHURST, Andrew, San Antonio Texas 78245 (US)
(74) Representative: Walker, Ross Thomson
(86) International application number: PCT/US2012/049598
(87) International publication number: WO 2013/020087

(56) References cited:
- WO-A1-2011/089527
- US-A1- 2002 142 355
- US-A1- 2005 152 912
- US-A1- 2007 065 913
- KREBBER A ET AL: "Reliable cloning of functional antibody variable domains from hybridomas and spleen cell repertoires employing a reengineered phage display system", JOURNAL OF IMMUNOLOGICAL METHODS, ELSEVIER SCIENCE PUBLISHERS B.V.,AMSTERDAM, NL, vol. 201, no. 1, 14 February 1997 (1997-02-14), pages 35-55, XP004050040, ISSN: 0022-1759, DOI: 10.1016/S0022-1759(96)00208-6
- SIBLER A-P ET AL: "In vivo biotinylated recombinant antibodies: high efficiency of labelling and application to the cloning of active anti-human IgG1 Fab fragments", JOURNAL OF IMMUNOLOGICAL METHODS, ELSEVIER SCIENCE PUBLISHERS B.V.,AMSTERDAM, NL, vol. 224, no. 1-2, 22 April 1999 (1999-04-22), pages 129-140, XP004165516, ISSN: 0022-1759, DOI: 10.1016/S0022-1759(99)00016-2
- AL-MRABEH A ET AL: "A fully recombinant ELISA using in vivo biotinylated antibody fragments for the detection of potato leafroll virus", JOURNAL OF VIROLOGICAL METHODS, ELSEVIER BV, NL, vol. 159, no. 2, 1 August 2009 (2009-08-01), pages 200-205, XP026159977, ISSN: 0166-0934, DOI: 10.1016/J.JVIROMET.2009.03.025 [retrieved on 2009-04-05]
- HOOGENBOOM H R ET AL: "MULTI-SUBUNIT PROTEINS ON THE SURFACE OF FILAMENTOUS PHAGE: METHODOLOGIES FOR DISPLAYING ANTIBODY (FAB) HEAVY AND LIGHT CHAINS", NUCLEIC ACIDS RESEARCH, OXFORD UNIVERSITY PRESS, GB, vol. 19, no. 15, 1 January 1991 (1991-01-01), pages 4133-4137, XP001019229, ISSN: 0305-1048
- LAURA J SHERWOOD ET AL: "Rapid assembly of sensitive antigen-capture assays for Marburg virus, using in vitro selection of llama single-domain antibodies, at biosafety level 4", JOURNAL OF INFECTIOUS DISEASES. JID, UNIVERSITY OF CHICAGO PRESS, CHICAGO, IL, vol. 196, no. Suppl 2, 15 November 2007 (2007-11-15), pages S213-S219, XP002673501, ISSN: 0022-1899, DOI: 10.1086/520586
- LAURA J. SHERWOOD ET AL: "Hapten Mediated Display and Pairing of Recombinant Antibodies Accelerates Assay Assembly for Biothreat Countermeasures", SCIENTIFIC REPORTS, vol. 2, 12 November 2012 (2012-11-12), XP055158833, DOI: 10.1038/srep00807
- BRIAN K. KAY ET AL.: ' SCREENING PHAGE-DISPLAYED COMBINATORIAL PEPTIDE LIBRARIES' METHODS. vol. 24, no. 3, 2001, pages 240 - 246, XP002257757

## Description

### FIELD OF INVENTION

The present inventions relate to the field of molecular biology tools and methods for diagnostic and therapeutic applications. Specifically, the inventions relate to the selection of peptides or proteins with desired properties, like specific binding affinity or greater stability or improved solubility, from vast numbers of variants. Further, the invention may be applied to the identification and characterization of any pair of affinity agent(s) including non-aminoacid motifs like synthetic aptamers and haptens, or other molecular configurations that are not derived from a nucleic acid-based system.

### BACKGROUND OF THE INVENTION

Phage technology involves engineering bacteriophages to generate proteins or peptides from a library of variants. The protein or peptide of interest can either be produced as soluble molecules or be displayed as coat protein fusions on the surface of a phage or phagemid particle. Some phage constructs (e.g. pHEN vectors) permit both the display of the desired protein on the surface of the phage and the soluble expression of the protein. But these phage constructs have to be transfected into different host cells to achieve the desired expression state of the protein. For example, a phage construct may produce the displayed form of the protein in a host cell expressing the suppressor tRNA supE and the soluble form of the protein in a non-supE host cell. This two-host cell system has several logistical and molecular disadvantages. It would be beneficial to work within a single host cell, and use a biomolecular switch to change the expression profile of the desired protein from the displayed form to the soluble form or vice-versa.

Phage technology is a powerful tool to study protein interactions with target molecules and it is useful in identifying epitopes, mimotopes, and other functional and accessible sites of target molecules. The technology is also useful in vaccine design and engineered phages can serve as vaccine delivery units. One of the most dominant applications of phage technology is to generate proteins or peptides, especially monoclonal antibodies, with affinity to molecules of interest. Once libraries have been mined for desired properties, like specific binding properties or greater stability profiles, the subsequent characterization of those binding agents or affinity agents becomes a time consuming and expensive process. Whereas therapeutic applications may rely on a single affinity agent, many diagnostic applications require two unique affinity reagents that bind the target molecule non-competitively. One is the captor or the affinity agent of interest that binds to the molecule of interest. The second, is the tracer, an affinity agent for the detection of the bound captor-molecule of interest complex. Some diagnostic applications may function using a single affinity agent as long as the antigen or molecule of interest is oligomeric (i.e. dimer or higher multimer). There is a need for agents and methods that screen for and utilize a single affinity agent as both the captor and the tracer. There is also a need for agents and methods that screen for and utilize unique affinity agents as captor and tracer. If reagents and methods could, be developed to satisfy these needs, it would enable rapid pairing of affinity agents in any circumstance, leading to increased efficiencies in time and resources.

KREBBER A et al in Journal of Immunological Methods 201 (1997), pages 35-37 discloses the cloning of a functional antibody variable domain from hybridomas and spleen cell repertoires employing a reengineered phage display system.

SIBLER A-P et al in Journal of Immunological Methods 224 (1999), pages 129-140 discloses in vivo biotinylated recombinant antibodies.

AL-MRABEH A et al in Journal of Virological Methods 159 (2009), pages 200-205 discloses a fully recombinant ELISA using in vivo biotinylated antibody fragments for the detection of potato leafroll virus.

### SUMMARY OF THE INVENTION

In the first aspect of the present invention there is provided a cell comprising:
a first nucleic acid comprising a first promoter and an expression cassette encoding a polypeptide of interest, a biotin substrate site, and a display protein, the expression cassette having a termination codon between the polypeptide of interest and the display protein; and
a second nucleic acid comprising a second promoter and a gene encoding a suppressor tRNA that recognizes the termination codon in the first nucleic acid.

In a further aspect of the present invention there is provided a method of generating biotinylated polypeptides of interest, the method comprising:
producing a cell comprising a nucleic acid comprising a promoter and an expression cassette encoding a polypeptide of interest, a biotin substrate site, and a display protein, and the expression cassette having a termination codon between the polypeptide of interest and the display protein;
incubating the cell under conditions sufficient for expression of the polypeptide of interest with the biotin substrate site; and
providing an agent capable of biotinylating the polypeptide of interest at the biotin substrate site.

In further aspect of the present invention there is provided a method of generating biotinylated polypeptides of interest, the method comprising:
producing a cell comprising
   a first nucleic acid comprising a first promoter and an expression cassette encoding a polypeptide of interest, a biotin substrate site, and a display protein, and the expression cassette having a termination codon between the polypeptide of interest and the display protein; and
   a second nucleic acid comprising a second promoter and a gene encoding a suppressor tRNA that recognizes the termination codon in the first nucleic acid;
incubating the cell under conditions sufficient for protein expression from the first promoter in the first nucleic acid and the second promoter in the second nucleic acid, thereby the polypeptide of interest with the biotin substrate site and the display protein is produced; and
providing an agent capable of biotinylating the polypeptide of interest at the biotin substrate site, thereby producing biotinylated polypeptides of interest with the display protein.

In a further aspect of the present invention there is provided A method of screening for a polypeptide of interest with affinity to a molecule of interest, the method comprising:
providing an array comprising biotin affinity agents;
producing biotinylated polypeptides comprising
   producing a cell comprising a nucleic acid comprising a promoter and an expression cassette encoding a polypeptide of interest, a biotin substrate site, and a display protein, and the expression cassette having a termination codon between the polypeptide of interest and the display protein ;
   incubating the cell under conditions sufficient for expression of the polypeptide of interest with the biotin substrate site; and providing an agent capable of biotinylating the polypeptide of interest at the biotin substrate site;
adding a cellular fraction comprising the biotinylated polypeptides to the array;
adding a molecule of interest to bind to the biotinylated polypeptides; adding the cellular fraction comprising the biotinylated polypeptides; and detecting the polypeptide of interest that exhibits binding affinity to the molecule of interest.

### BRIEF DESCRIPTION OF THE DRAWINGS

So that the manner in which the above-recited features, aspects and advantages of the invention, as well as others that will become apparent, are attained and can be understood in detail, more particular description of the invention briefly summarized above can be had by reference to the embodiments thereof that are illustrated in the drawings that form a part of this specification. It is to be noted, however, that the appended drawings illustrate only some embodiments of the invention and are, therefore, not to be considered limiting of the invention's scope, for the invention can admit to other equally effective embodiments.

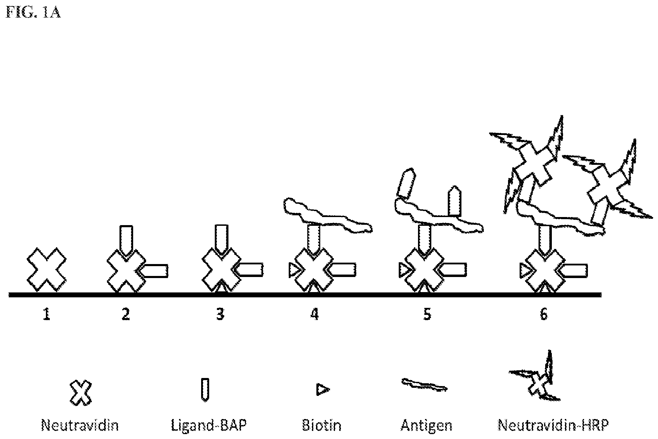

**FIG. 1A** is an illustration of the rapid affnity agent pairing method.

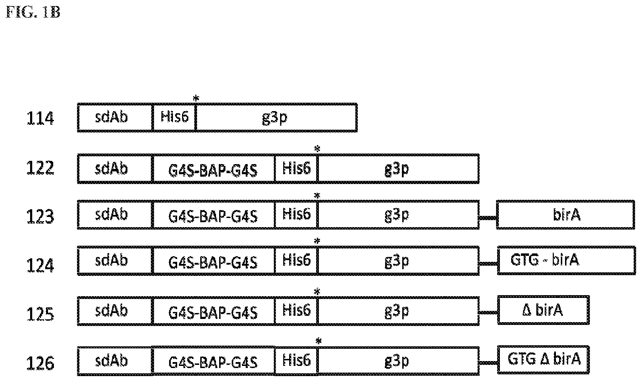

**FIG. 1B** is an illustration of the nucleic acid compositions used to establish the rapid pairing system.

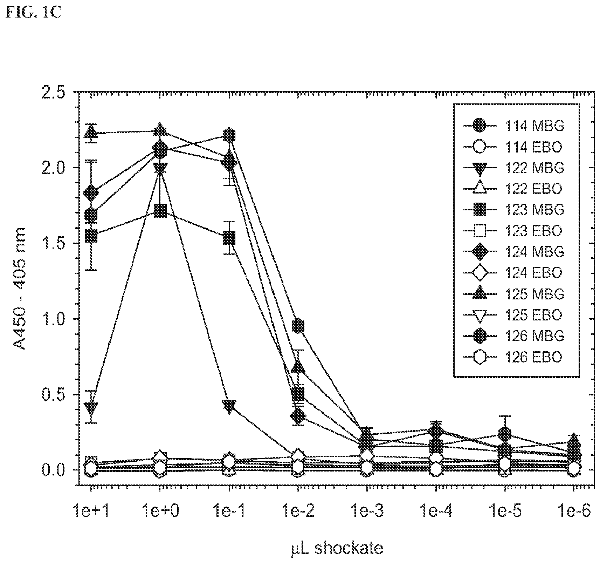

**FIG. 1C** is a graph that shows the rapid affinity agent pairing system using the biotinylated antibodies as captors and phage displayed antibodies as tracers. The x axis indicates the volume of shockate used and the y axis indicates the absorbance units from measuring the product of the horseradish peroxidase (HRP) activity. The HRP here is conjugated to an anti-M13 monoclonal antibody.

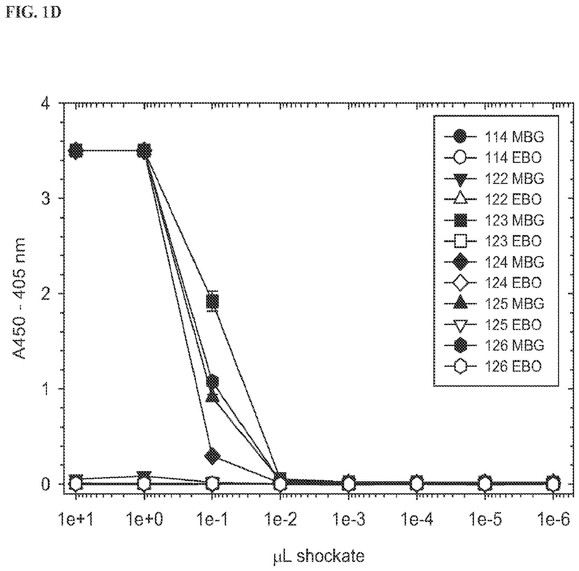

**FIG. 1D** is a graph that shows the rapid affinity agent pairing system using the biotinylated antibodies as both captors and tracers. The x axis indicates the volume of shockate used, and the y axis indicates the absorbance units from measuring the product of the horseradish peroxidase activity. The HRP here is conjugated to neutravidin.

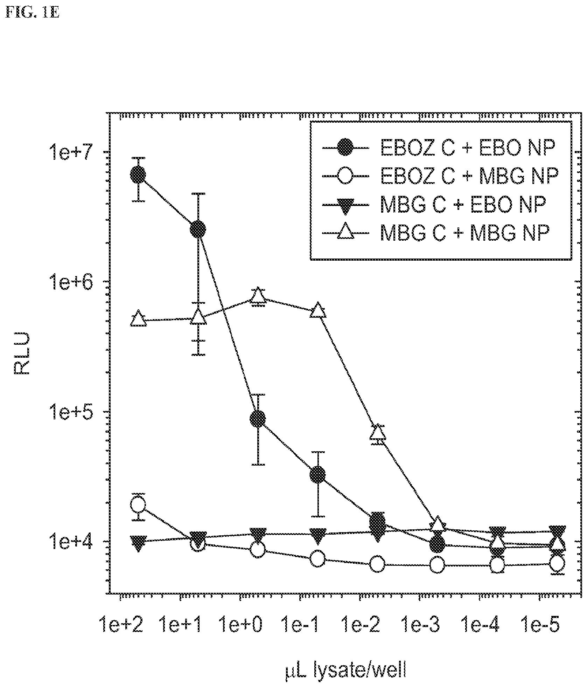

**FIG. 1E** is a graph showing that recombinant Marburg and Ebola Zaire virus nucleoprotein mimics are acceptable surrogates for developing sdAbs against the respective viruses. The x axis indicates the volume of crude *E.coli* lysate expressing the recombinant nucleoprotein, and the y axis indicates a measure of luminescence derived from the enzymatic activity of the alkaline phosphatase fusion to the sdAb.

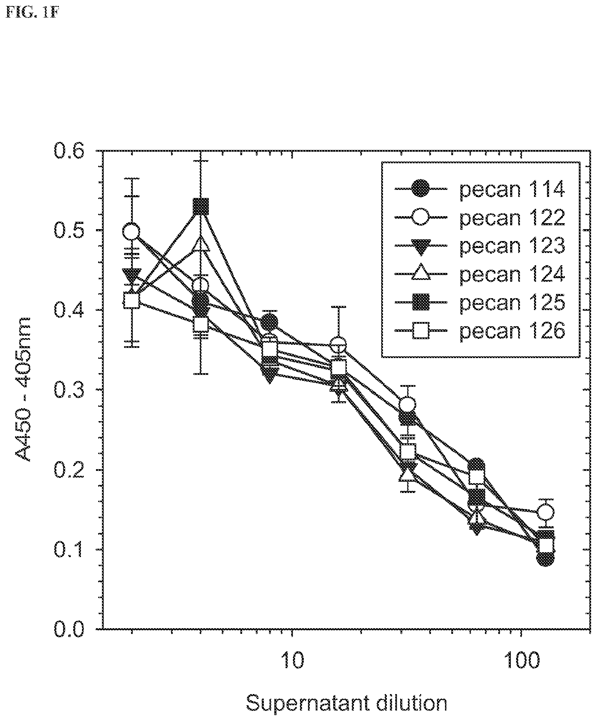

**FIG. 1F** is a graph that shows the effect of the birA mutations on the positive phage production levels, following the expression of various vectors in XL-1 Blue host and analyzed by a phage display ELISA. The x axis indicates the dilution of supernatant containing the phage, and the y axis indicates the absorbance units from measuring the product of the horseradish peroxidase activity. The HRP here is conjugated to an anti-M13 monoclonal antibody.

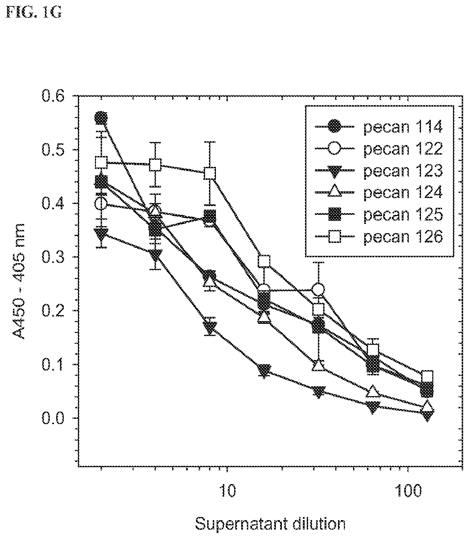

**FIG. 1G** is a graph that shows the effect of the birA mutations on the positive phage production levels, following the expression of various vectors in HBV88 host and analyzed by a phage display ELISA. The x axis indicates the dilution of supernatant containing the phage, and the y axis indicates the absorbance units measuring the product of the horseradish peroxidase activity. The HRP here is conjugated to an anti-M13 monoclonal antibody.

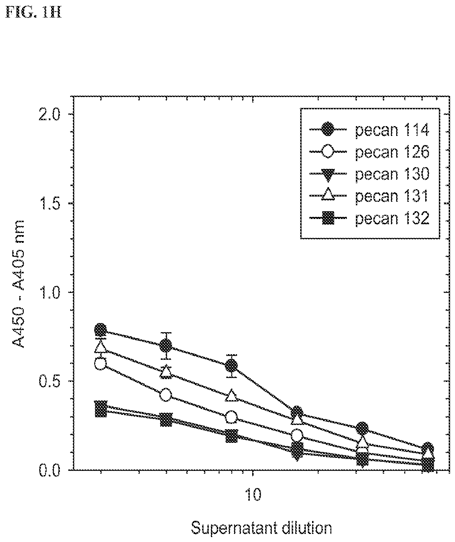

**FIG. 1H** is a graph that shows the effect of the variations in linkers, between the BAP sequence and the sdAb/ His6 tag, on the positive phage production levels, following the expression of various vectors in XL-1 Blue host and analyzed by a phage display ELISA. The x axis indicates the dilution of supernatant containing the phage, and the y axis indicates the absorbance units measuring the product of the horseradish peroxidase activity. The HRP here is conjugated to an anti-M13 monoclonal antibody.

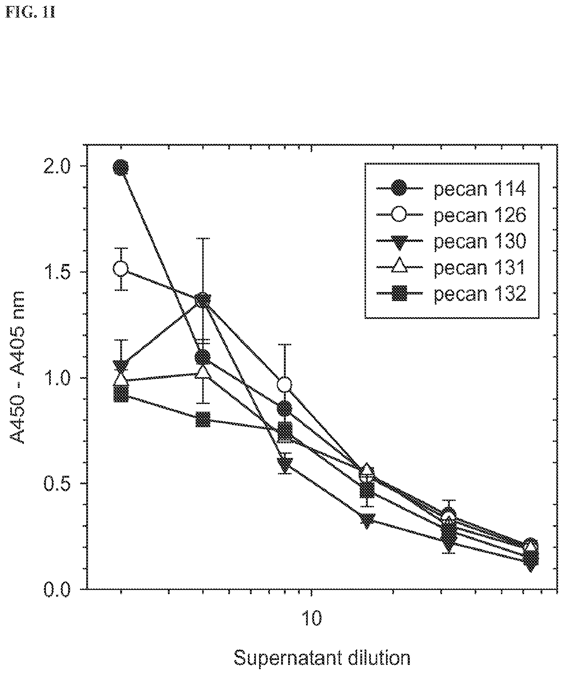

**FIG. 1I** is a graph that shows the effect of the variations in linkers, between the BAP sequence and the sdAb/ His6 tag, on the positive phage production levels, following the rescue of various vectors in the HBV88 host in display mode and analyzed by a phage display ELISA. The x axis indicates the dilution of supernatant containing the phage, and the y axis indicates the absorbance units measuring the product of the horseradish peroxidase activity. The HRP here is conjugated to an anti-M13 monoclonal antibody.

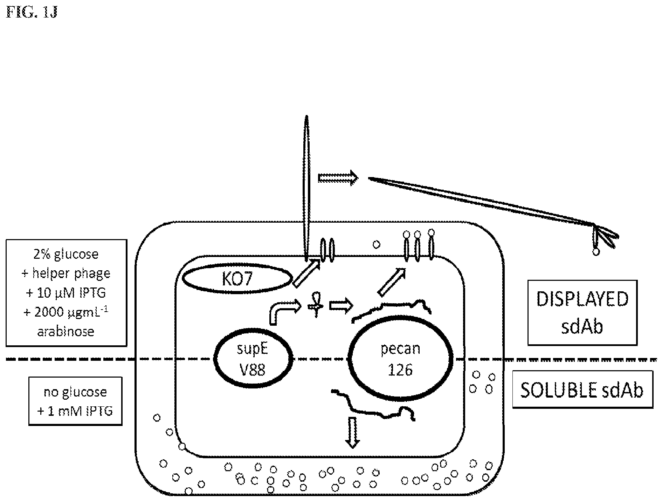

**FIG. 1J** is a schematic drawing of the method of switching the expression profile of the polypeptide of interest from the soluble form to the displayed form by inducing the expression of the suppressor tRNA.

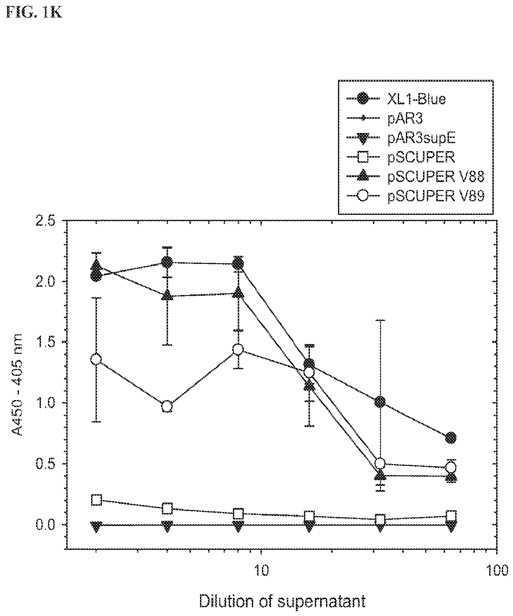

**FIG. 1K** is a graph that shows the expression profile of the antibodies as displayed proteins when the suppressor tRNA constructs are present in different expression vectors in HBV88. The x axis indicates the dilution of supernatant containing the phage, and the y axis indicates the absorbance units measuring the product of the horseradish peroxidase activity. The HRP here is conjugated to an anti-M13 monoclonal antibody.

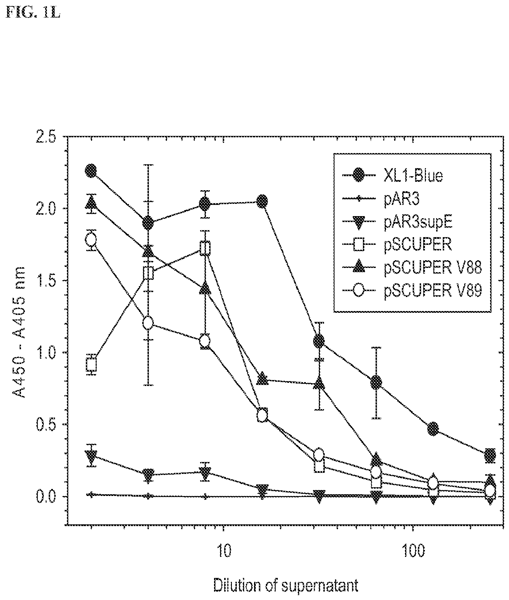

**FIG. 1L** is a graph that shows the expression profile of the antibodies as displayed proteins when the suppressor tRNA constructs are present in different expression vectors in DH10BF' host cells. The x axis indicates the dilution of supernatant containing the phage. and the y axis indicates the absorbance units measuring the product of the horseradish peroxidase activity. The HRP here is conjugated to an anti-M13 monoclonal antibody.

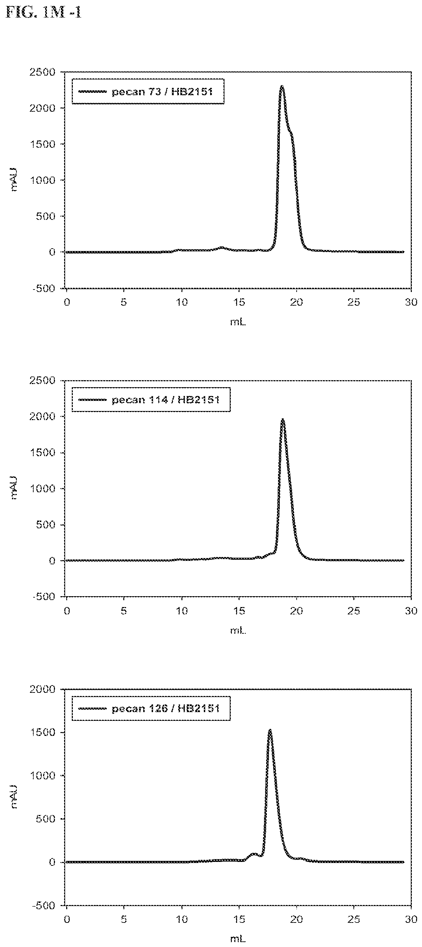

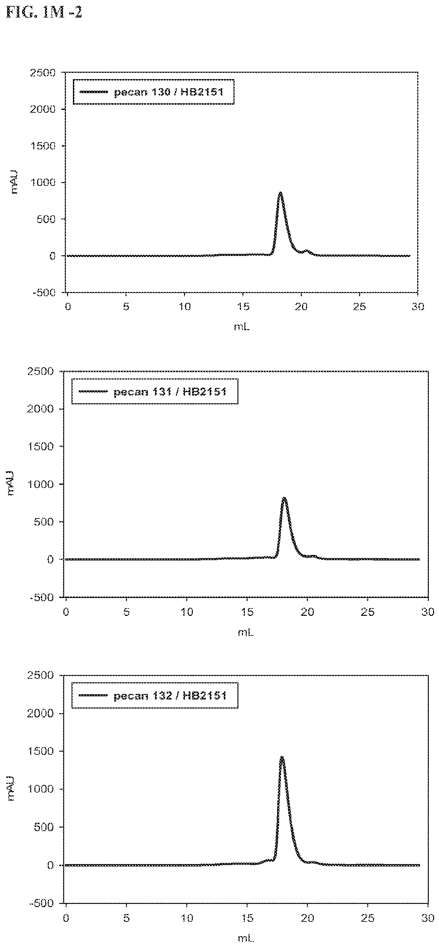

**FIG. 1M-1** **and** **-2** are the Fast Protein Liquid Chromatography (FPLC) gel filtration evaluations of proteins produced from vectors, containing the variations in linkers between the BAP sequence and the sdAb/ His6 tag, and expressed in a conventional non-suppressor host strain HB2151. The x axis indicates the elution volumes from the FPLC, and the y axis indicates the ultraviolet absorbance units for the protein contained in the fraction.

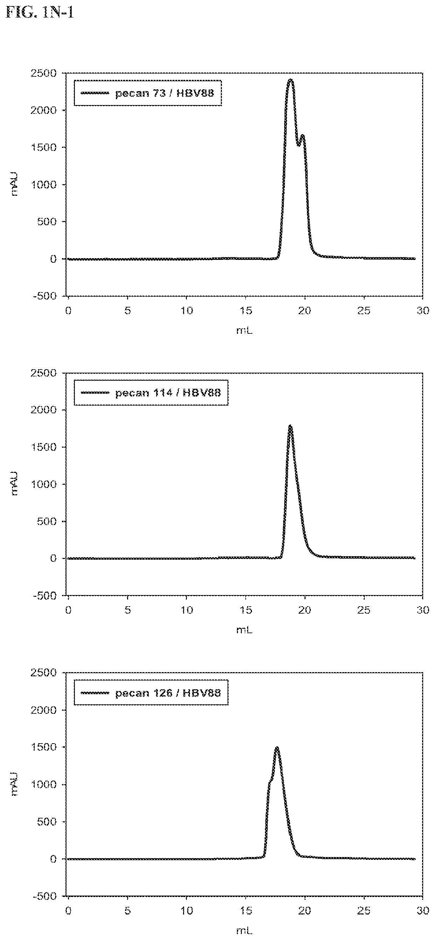

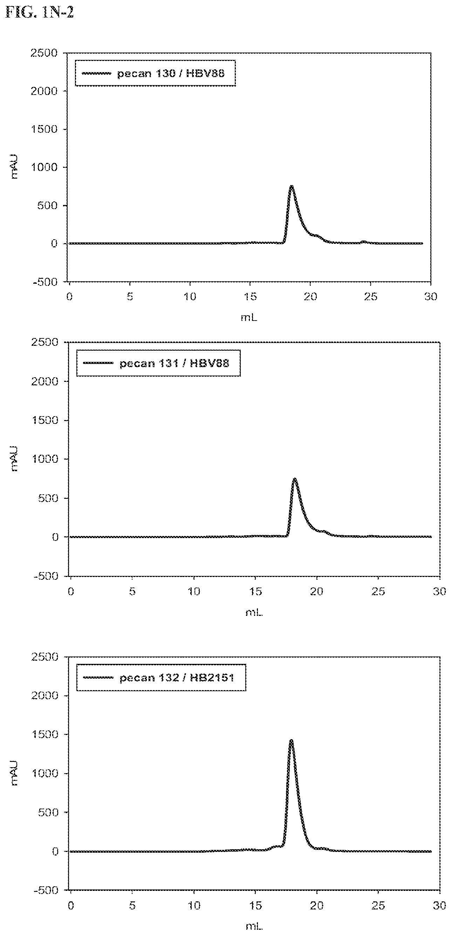

**FIG. 1N-1** **and** **-2** are the FPLC evaluations of proteins produced from vectors, containing the variations in linkers between the BAP sequence and the sdAb/ His6 tag, and expressed in the conditional suppressor strain HBV88. The x axis indicates the elution volumes from the FPLC, and the y axis indicates the absorbance units for the protein contained in the fraction.

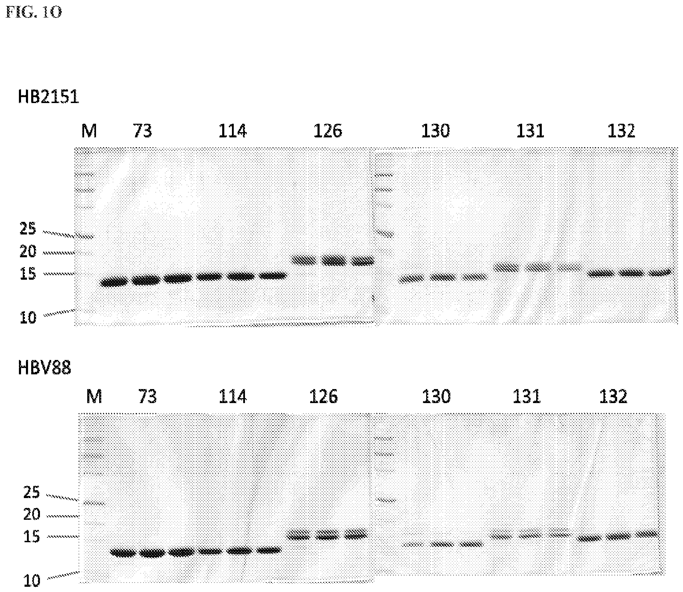

**FIG. 1O** is a picture of the polyacrylamide gel analysis of the proteins produced from vectors, containing the variations in linkers between the BAP sequence and the sdAb/ His6 tag. The upper half of the picture shows expression in the conventional non-suppressor host strain HB2151, and the lower half of the picture shows expression in the conditional suppressor strain HBV88.

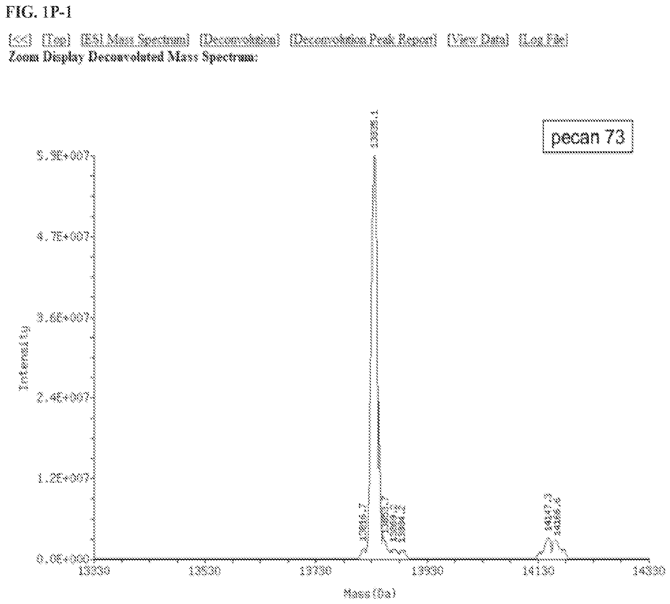

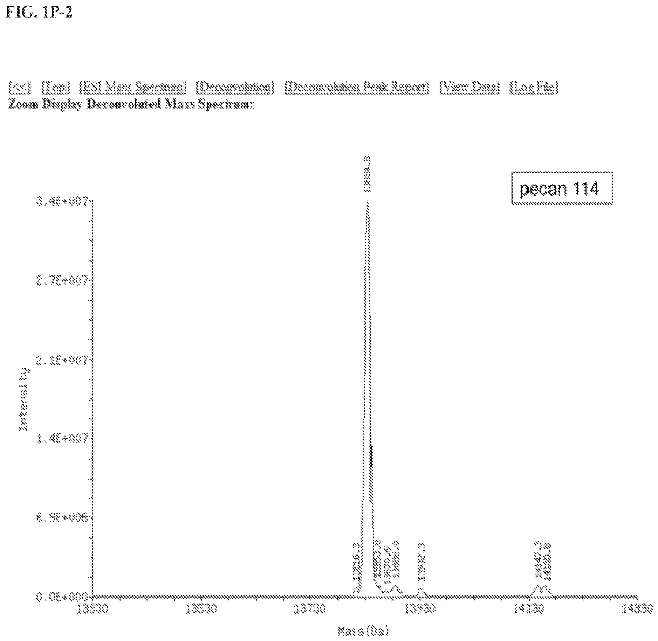

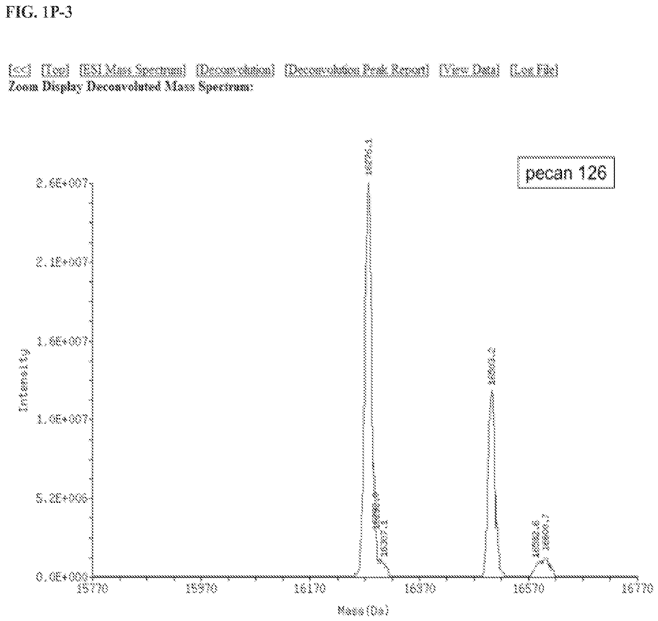

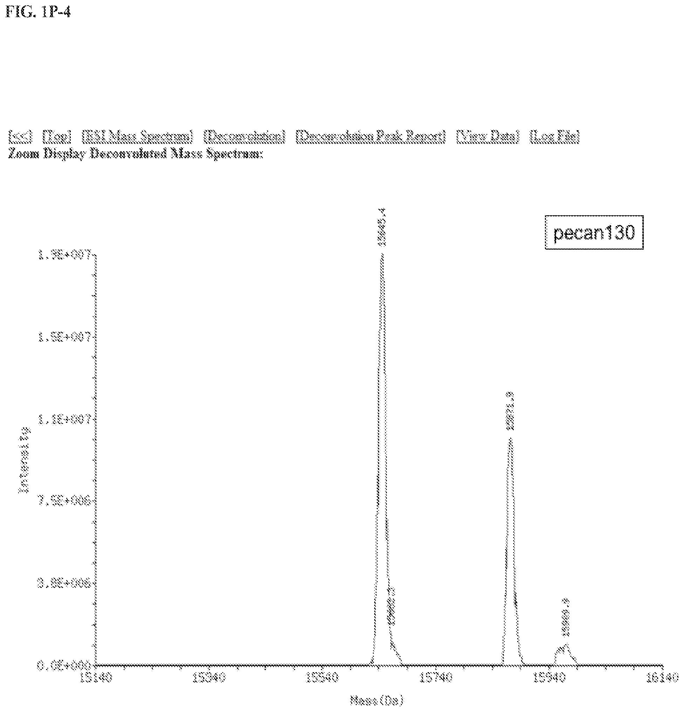

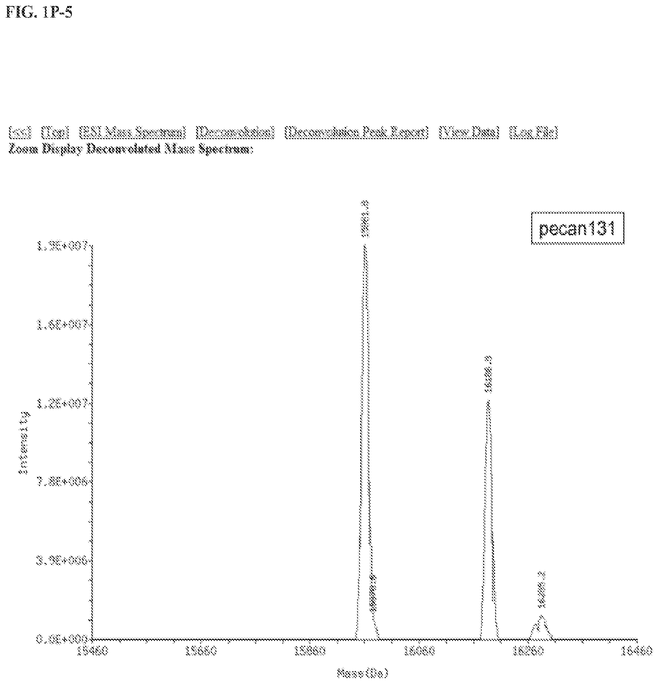

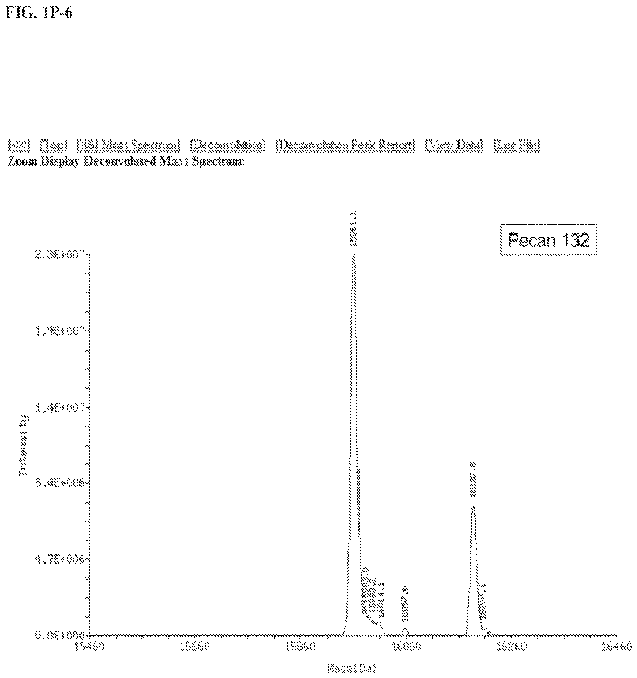

**FIG. 1P-1****,** **-2****,** **-3****,** **-4****,** **-5** **and** **-6** are the mass-spectrometry analysis of the peak fractions of the proteins produced from vectors, containing the variations in linkers between the BAP sequence and the sdAb/ His6 tag.

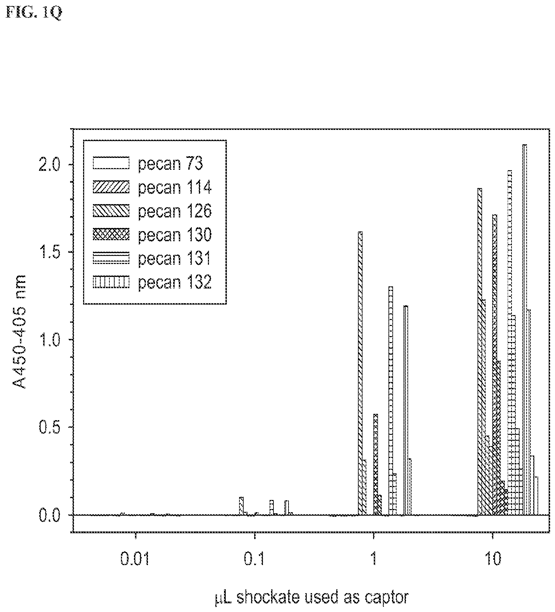

**FIG. 1Q** is a graph showing that the single species of sdAb produced within the host cell HBV88, used as both captor and tracer affinity agent, recognizes a polyvalent antigen, demonstrated by the highly specific ELISA signals on positive control surrogate antigen. The x axis indicates the volume of the shockate used as captor, and the y axis indicates the absorbance units measuring the product of the horseradish peroxidase activity. 10, 1,01 and 0.01 µL of the shockates were used either as captor (denoted on the x-axis) or as tracers (left to right bars in each vector set). The HRP here is conjugated to neutravidin.

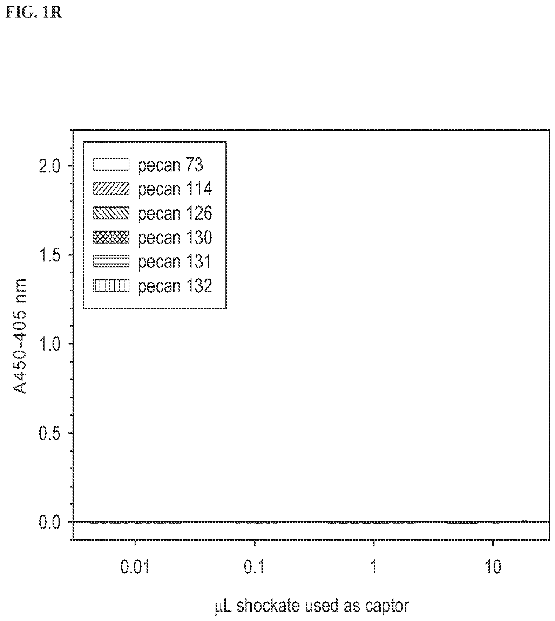

**FIG. 1R** is a graph showing that the single species of sdAb produced within the host cell HBV88, used as both captor and tracer affinity agent, recognizes only the positive control surrogate antigen **(****FIG. 1Q****)** but not negative control surrogate antigen. The x axis indicates the volume of the shockate used as captor, and the y axis refers to the absorbance units measuring the product of the horseradish peroxidase activity. The HRP here is conjugated to neutravidin.

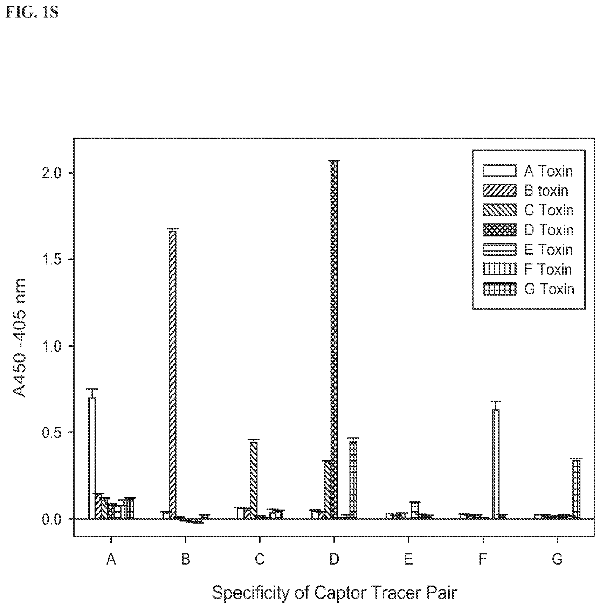

**FIG. 1S** is a graph that shows the specificity of the rapid affinity agent pairing of 7 pairs of heterologous sdAb to the 7 serotypes of botulinum neurotoxin (BoNT). The x axis indicates the seven different pairs individually challenged with each toxin serotype, and the y axis refers to the absorbance units measuring the product of the horseradish peroxidase activity. The HRP here is conjugated to neutravidin.

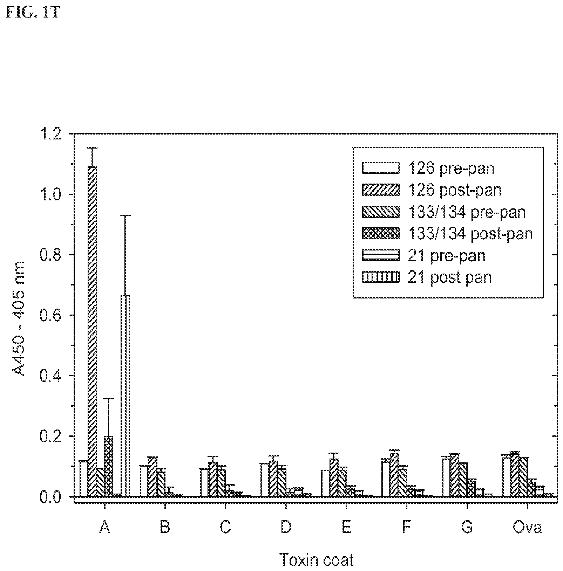

**FIG. 1T** is a graph that shows that both the standard display format (pecan21) armed with rapid affinity agent pairing ability (pecan126) and the similarly armed transdisplay system (pecan133/134) can generate single domain antibodies specific for their desired target (BoNT serotype A) when screening from libraries of 1e⁺⁸ and 1e⁺⁹ respectively using a standard panning method. The x axis indicates the antigens used to coat the plate, and the y axis refers to the absorbance units from measuring the product of the horseradish peroxidase activity. The HRP here is conjugated to an anti-M13 monoclonal antibody.

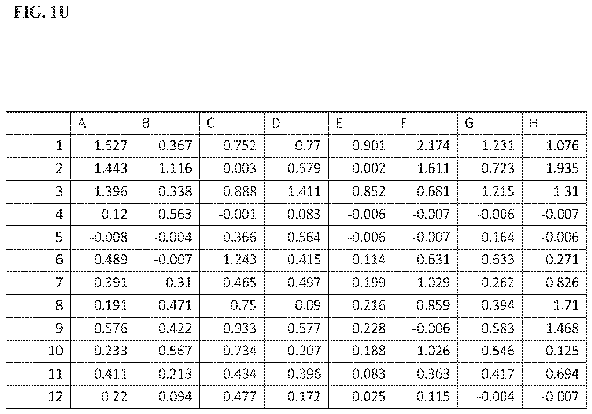

**FIG. 1U** is a dataset for monoclonal phage ELISA of clones from HBV88+ pecan126 screened on plate coated with botulinum neurotoxin A (BoNT A).

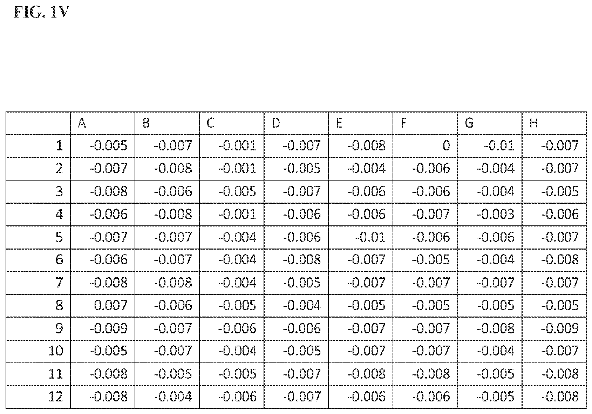

**FIG. 1V** is the dataset for monoclonal phage ELISA of the same clones from HBV88+ pecan126 used above, screened on plate coated with ovalbumin.

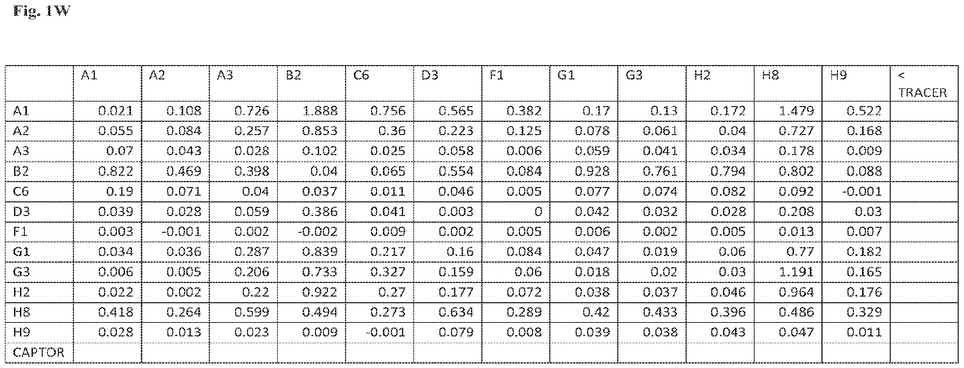

**FIG. 1W** is the dataset for positive clones identified from pecan126/HBV88 heptaplex BoNT immune library, used as captor and tracer pairs in a checkerboard fashion, capable of recognizing 1 µg/mL BoNT serotype A in solution.

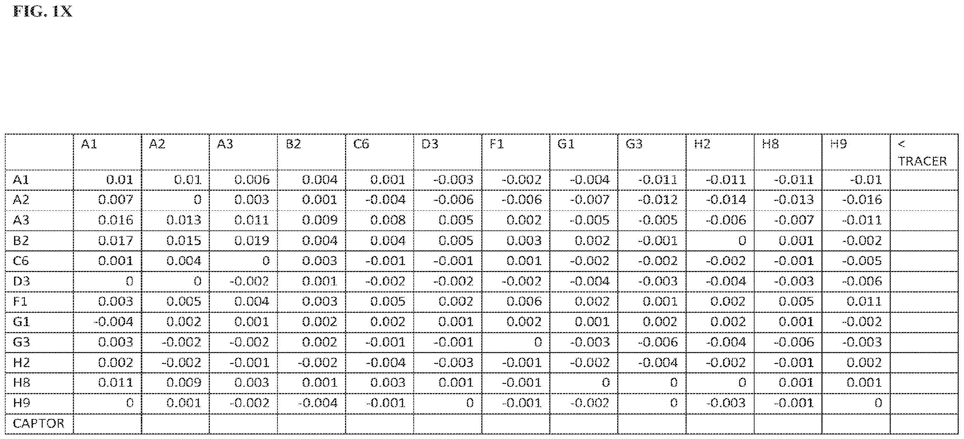

**FIG. 1X** is the dataset for the negative control of **FIG. 1W****,** using the same clones from the pecan126/HBV88 heptaplex BoNT immune library on 1 µg/mL ovalbumin in solution.

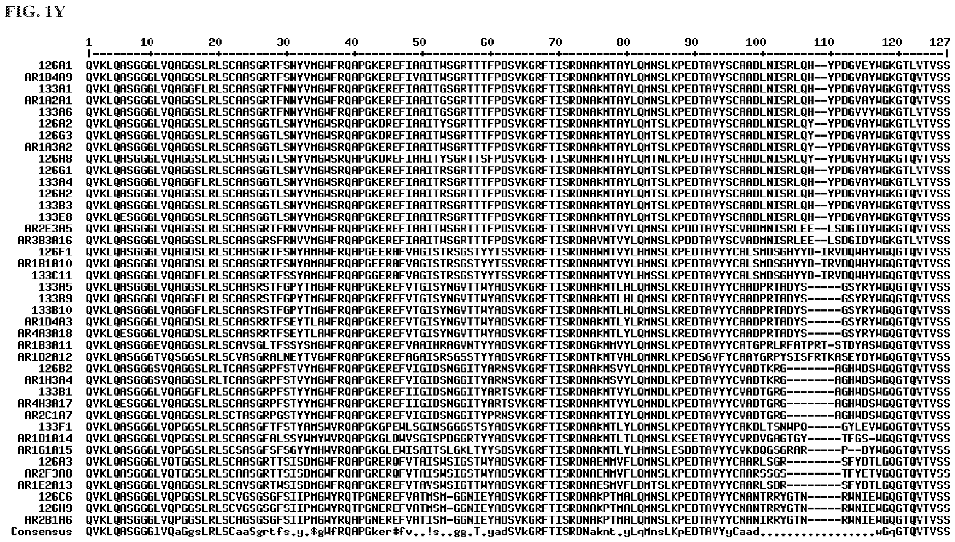

**FIG. 1Y** is the amino acid sequence alignment of the sdAb clones selected using pecan126 within HBV88 (126) and pecan133 + pecan134 within HB1251 (133) aligned with the original anti-BoNT A clones from conventional panning.

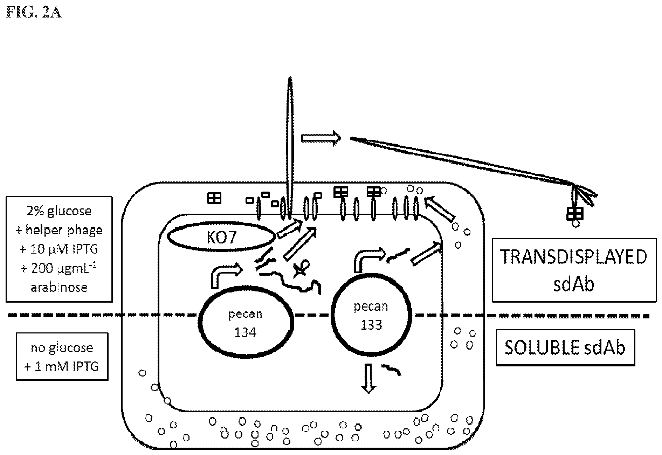

**FIG. 2A** is a schematic drawing of the transdisplay system, wherein the display protein and the polypeptide of interest are encoded by separate expression cassettes in the same host cell.

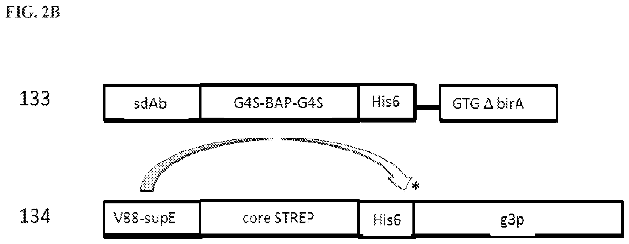

**FIG. 2B** is an illustration of the nucleic acid compositions that may be used to establish the trans-display system.

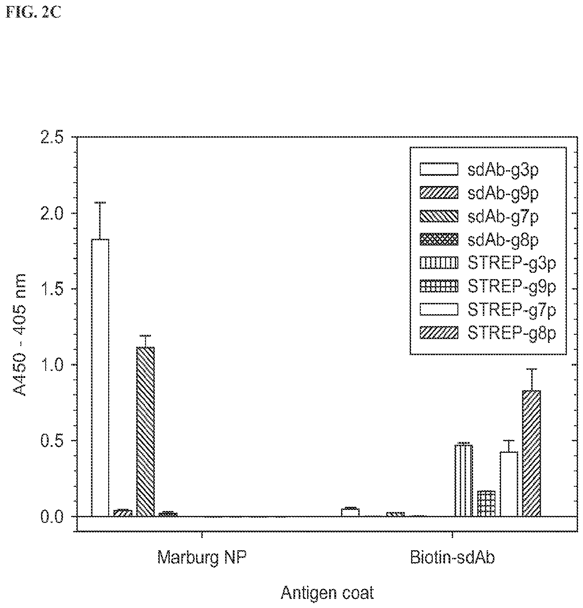

**FIG. 2C** is a graph showing the ELISA capture of sdAb (sdAb) or core streptavidin (STREP) displayed on the various coat proteins of M13 phage.

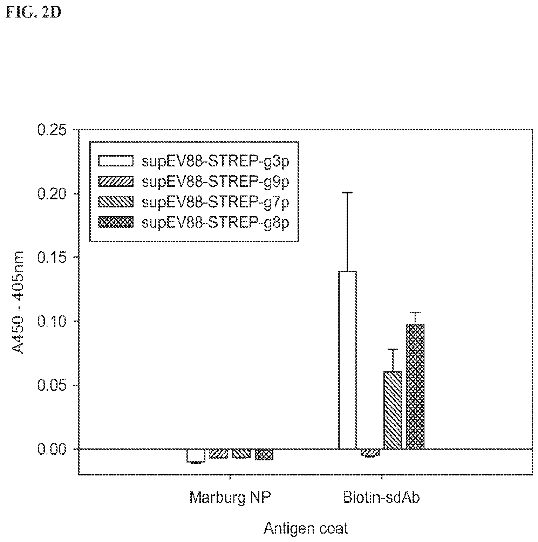

**FIG. 2D** is a graph showing the expression of streptavidin when using pSCUPER backbone vectors having the supE exchanged for supE- streptavidin-platform gene fusions.

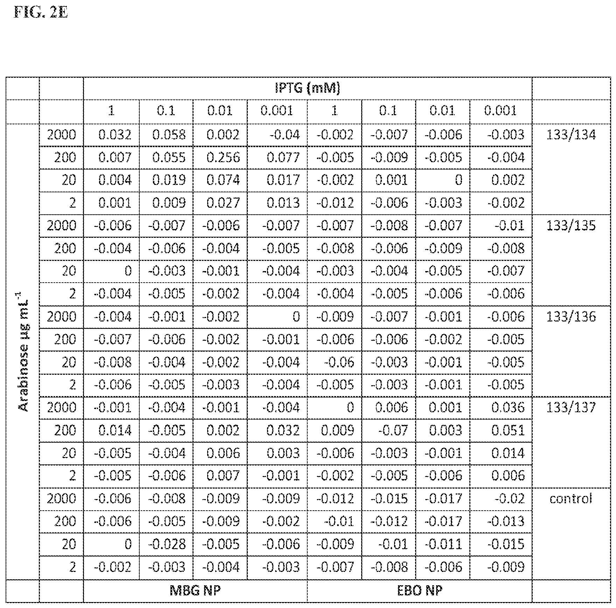

**FIG. 2E** is a table demonstrating the checker-board optimization of arabinose (2000 to 2 µgmL-1) and IPTG concentrations (1000 to 1 µM) for transdisplay in HB2151 bearing supEV88-streptavidin platforms with pecan133. Signal here indicates measurement of the binding of phage-displayed sdAb to the positive Marburg antigen, measured indirectly as horseradish peroxidase activity. The HRP here is conjugated to an anti-M13 monoclonal antibody.

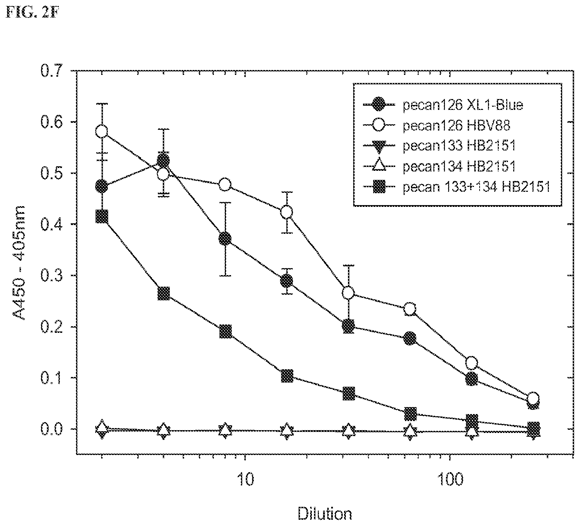

**FIG. 2F** is a graph that shows that the transdisplay system can generate displayed antibodies against the Marburg virus nucleoprotein. The x axis indicates the dilution of supernatant containing the phage, and the y axis indicates the absorbance units measuring the product of the horseradish peroxidase activity. The HRP here is conjugated to an anti-M13 monoclonal antibody.

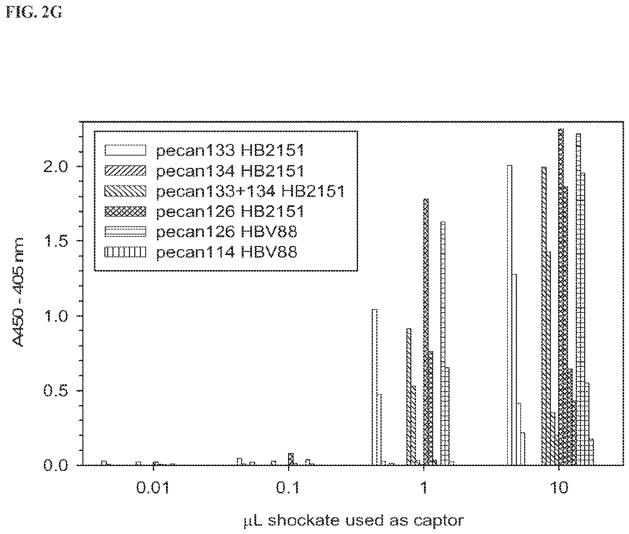

**FIG. 2G** is a graph showing that the transdisplay system can generate soluble biotinylated single domain antibodies and that these antibodies can function as captors and tracers in the rapid ligand pairing system to detect positive control antigen. The x axis indicates the volume of sdAbs-containing shockate used, and the y axis refers to the absorbance units measuring the product of the horseradish peroxidase activity. 10, 1,01 and 0.01 µL of the shockates were used either as captor (denoted on the x-axis) or as tracers (left to right bars in each vector set). The HRP here is conjugated to neutravidin.

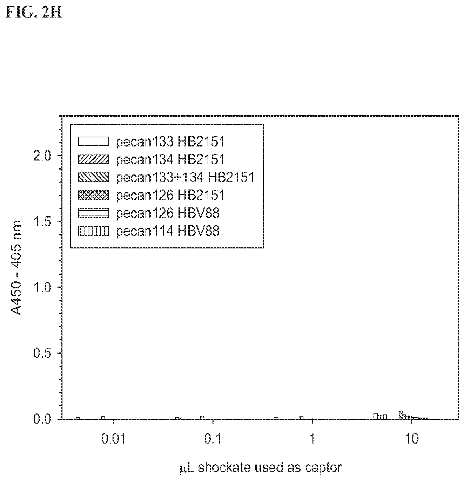

**FIG. 2H** is a graph showing that the transdisplay system can generate specific soluble biotinylated single domain antibodies which do not recognize the negative control antigen.

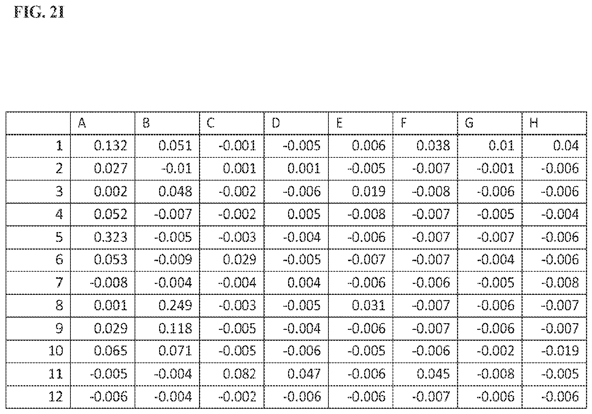

**FIG. 2I** is the dataset of the monoclonal phage ELISA signals from 96 clones of pecan133/134 immune heptaplex library on a plate coated with botulinum neurotoxin A (BoNTA).

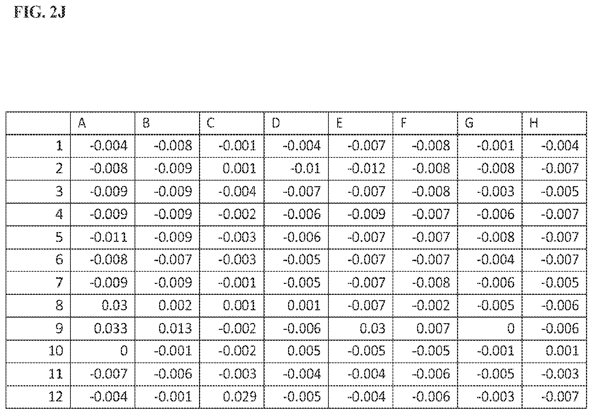

**FIG. 2J** is the dataset for the monoclonal phage ELISA signals from the 96 clones of pecan133/134 immune heptaplex library from **FIG. 2I** but on a plate coated with ovalbumin.

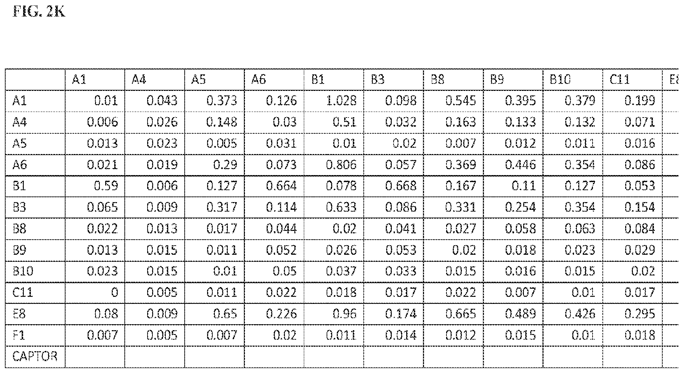

**FIG. 2K** is dataset obtained showing positive sdAb clones that were identified following selection and screening from pecan133/134 retrofitted heptaplex BoNT immune library can be paired in a checkerboard fashion to recognize BoNT A in solution.

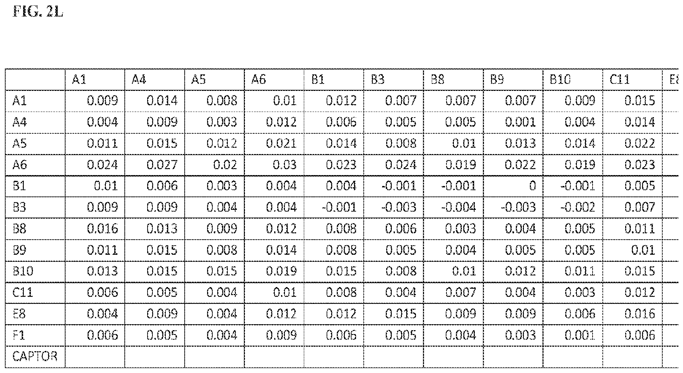

**FIG. 2L** is dataset obtained showing the lack of background signals when negative control antigen was used to identify sdAb clones.

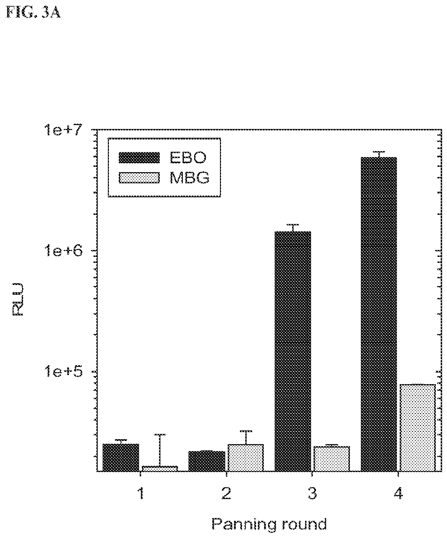

**FIG. 3A** is a graph showing the enrichment of polyclonal phage specific for Ebolavirus Zaire (EBO) rather than Marburgvirus Musoke (MBG) through four rounds of panning a semi-synthetic sdAb library (ie. no bias to a given antigen/target) on purified Ebolavirus Zaire.

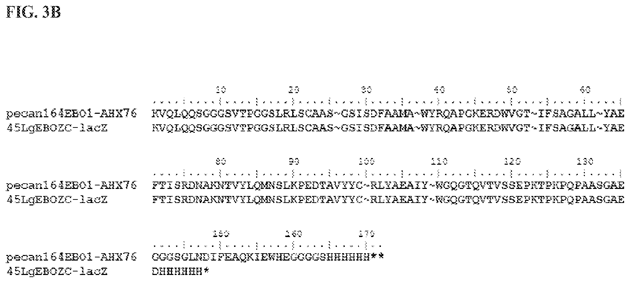

**FIG. 3B** is the amino acid sequence alignment of the anti-Ebolavirus Zaire clone isolated from transdisplay of the retrofitted single pot library, compared with the original sequence isolated via conventional panning.

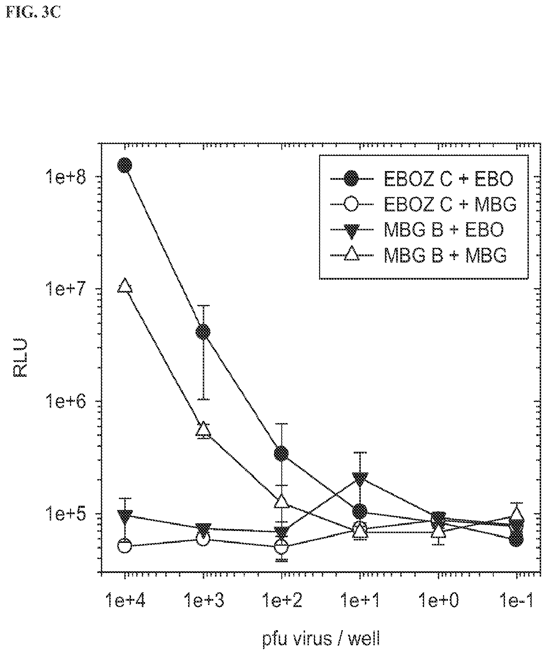

**FIG. 3C** is a graph showing the single sdAb clone within HB2151+pecan134 (EBOZ C) produced as shockate for captor and phage as tracer to generate an Ebolavirus Zaire (EBO) specific assay, while MBGB captor and phage from HB2151+pecan134/133 served to confirm Marburgvirus (MBG) control was present. The x axis denotes titration of either Ebolvirus or Marburgvirus, while y axis is absorbance due to horseradish peroxidase colorimetric activity. Here the peroxidase is conjugate to an anti-M13 antibody.

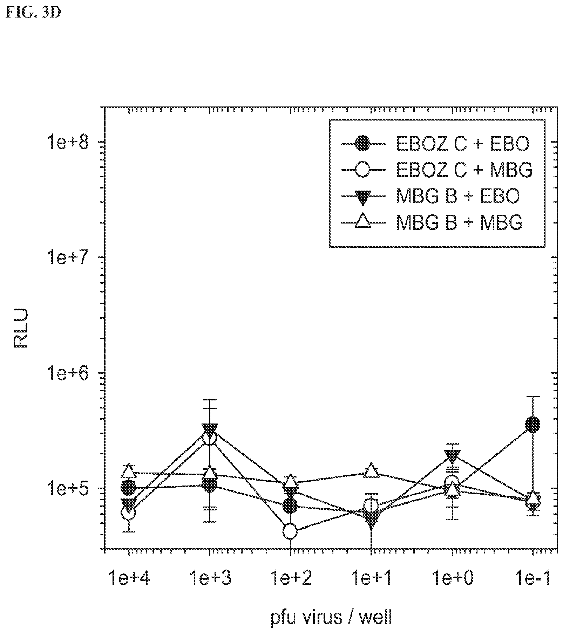

**FIG. 3D** is a graph showing that at low virus numbers the system can struggle for sensitivity without phage amplification.

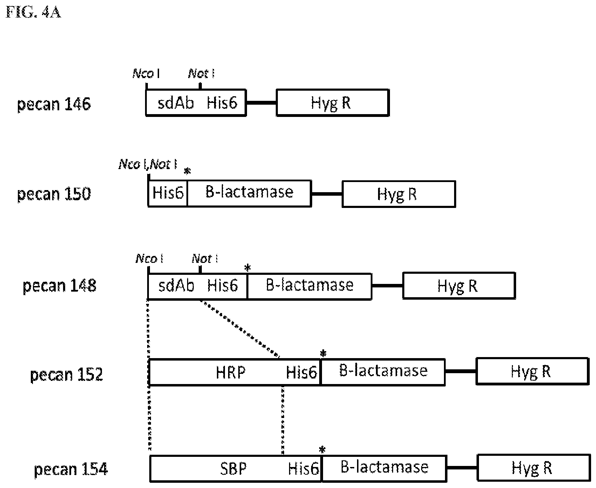

**FIG. 4A** is an exemplary illustration of the some vectors that may be assembled to select for improved soluble protein production. A basal level of β-lactamase expression afforded by the sdAb fusion (pecan 148) provides an estimate of the basal expression level of a protein of interest must achieve in order to become expressed at equivalent soluble levels in pecan148 HRP and pecan148 SBP.

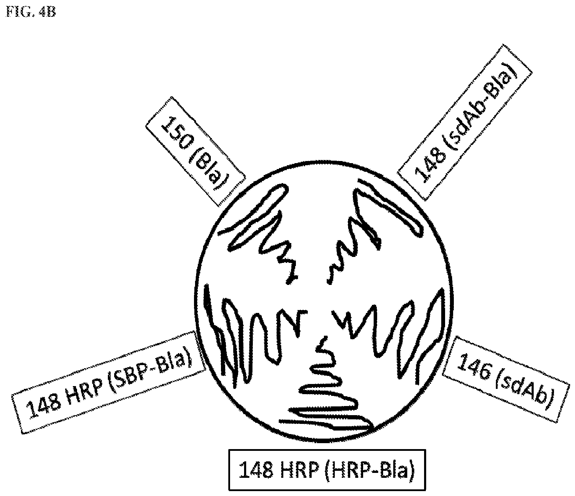

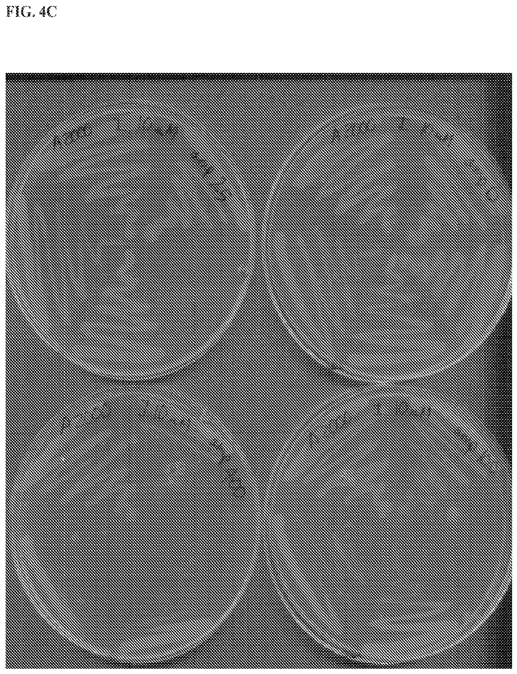

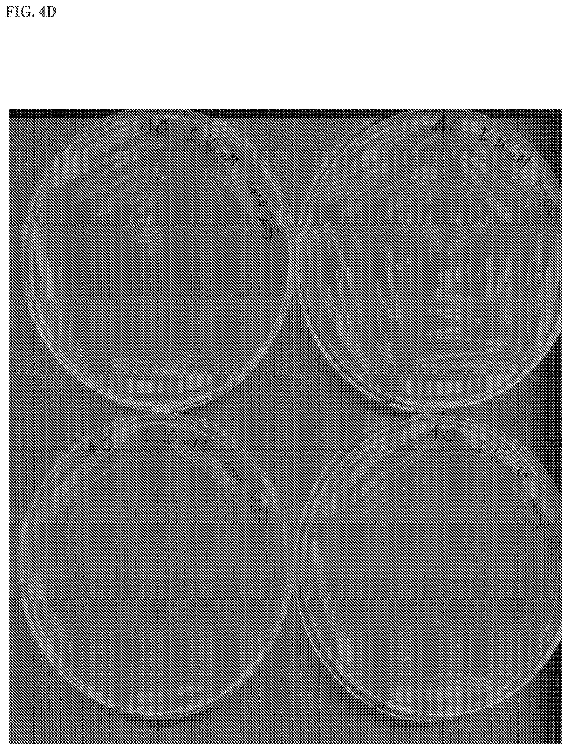

**FIGS. 4B-D** provide a demonstration of the method to select for enhanced soluble expression. **FIG. 4B** is an illustration of the vector construct contained in the host cells in each of the five sections of the plate. **FIG. 4C** is a picture that shows cells growing in the presence of arabinose 2000 at different concentrations of ampicillin and **FIG. 4D** is a picture that shows cells growing in the absence of arabinose and at different concentrations of ampicillin.

### DETAILED DESCRIPTION OF THE INVENTION

Before describing the embodiments of the present inventions in detail, several terms used in the context of embodiments of the present inventions will be defined. In addition to these terms, others are defined elsewhere in the specification, as necessary. Unless otherwise expressly defined herein, terms of art used in this specification will have their art-recognized meanings.

To more readily facilitate an understanding of the invention, the meanings of terms used herein will become apparent from the context of this specification in view of common usage of various terms and the explicit definitions provided below.

As used herein, the terms "comprising," "containing," "including," and "such as" are used in their open, non-limiting sense.

A "nucleic acid" or a "nucleic acid composition" means any genetic element, capable of serving as a vehicle for genetic transfer, expression, or replication of a polynucleotide of choice in a cell. The genetic element may also be functional in terms of expressing polypeptide(s) in a cell-free system as is the case during *in vitro* transcription or translation reactions, and may be a circular replication competent motif or a linear expression cassette e.g. PCR product. A nucleic acid composition may exist as a single polynucleotide or as two or more separate polynucleotides. For example, without limitation, a nucleic acid composition may be a vector, a plasmid, phagemid, or a cosmid or it may be capable of stable integration into the host cell genome. A nucleic acid composition may be a phage expression vector. A nucleic acid composition may be capable of replication in eukaryotic cells or prokaryotic cells or both. It may be present as a single copy or in multiple copies inside a cell. Examples of useful nucleic acid compositions that can be modified for use in the present invention include, but are not limited to, the expression vectors - pecan series, pHEN series, pUC series, pAK series, pET series, and pBAD series. The pecan series of vectors are proprietary vectors developed at the Texas Biomedical Research Institute. An embodiment may include one or more genes inserted into an expression vector, in proper orientation and in proximity to a promoter such that under proper conditions, expression of the polynucleotide of choice can be directed in an appropriate host cell. A nucleic acid composition may comprise at least one origin of replication and may also comprise a gene for a marker by which it can be identified or selected when inserted into a host cell. Useful markers are well known in the art and include for example, without limitations, markers that confer resistance to antibiotics, colorigenic or fluorogenic properties. The choice of a nucleic acid composition will depend on the properties of the host cell and the desired properties of the polynucleotide of interest.

A "gene" refers to a nucleic acid sequence that comprises the coding sequences necessary for the production of a RNA or a polypeptide or their precursors. A gene may include regulatory sequences preceding or following the coding sequence. The term gene encompasses both the cDNA and genomic forms of the gene.

A "promoter" means a polynucleotide sequence in a nucleic acid composition that controls transcription of a nucleic acid sequence to which it is operably linked. A promoter may include signals for RNA polymerase binding and transcription initiation. The promoters used will be functional in the cell type of the host cell in which expression of the selected sequence is contemplated. Some promoters including constitutive, inducible and repressible promoters are well known in the art, and are available from a variety of different sources. A promoter is usually located upstream of an expression cassette with the direction of transcription equivalent to the desired direction of translation of the polypeptide. A promoter is typically a sequence or sequences with affinity for an RNA polymerase sufficient to induce binding that is required for transcriptional initiation. A promoter may be repressible, inducible or constitutive. A repressible promoter's rate of transcription decreases in response to a repressing agent. A constitutive promoter's rate of transcription is not specifically regulated, though it can vary under the influence of general metabolic conditions. An inducible promoter's rate of transcription increases in response to an inducing agent. Examples of such promoters with their inducing agents are, without limitations, IPTG-inducible lac promoter, tetracycline-inducible T7 promoter, arabinose-inducible promoters, salt-inducible promoters, temperature-inducible promoters. A promoter may be able to drive a dynamic range of transcription levels by responding to the changes in the concentration of the inducer. When two or more expression cassettes or nucleic acid compositions are present, different promoters may be used to drive different expression profiles from the expression cassettes or nucleic acid compositions.

An "expression cassette" means a polynucleotide construct that contains coding sequences for one or more proteins that may be operably linked to a promoter sequence. An expression cassette may comprise other transcriptional regulatory sequences to direct proper transcription of the coding sequence into RNA. An expression cassette may also comprise any of a variety of translation regulatory sequences that may be necessary or desired to direct proper translation of the RNA in the intended host cell. The expression cassette is part of a nucleic acid composition and contains at least one gene that may be expressed by *in vitro* expression systems. The expression cassette is part of a nucleic acid composition and contains at least one gene that may be expressed by the host cell. The expression cassette may include other regulatory sequences including, but are not limited to, an initiation codon for translation start, a termination codon for ending translation, an RNA splice site, a transcriptional termination site, and a polyadenylation site. The expression cassette may contain the gene sequence for a protein of interest. An expression cassette may contain coding sequences for a tag or a post-translational modification site. An example of a post-translational modification site, without limitation, is the sequence coding for a biotin substrate site. The expression cassette may contain a linker between the gene sequences for the polypeptide of interest and the post-translational modification site. The expression cassette may also encode a protein that enables the post-translational modification of the protein. For example, without limitations, the expression cassette may encode a biotinylating agent, like a biotin ligase that adds a biotin at the biotinylation substrate site of the polypeptide of interest.

A nucleotide composition or a sequence "encoding" a polypeptide or a gene means a nucleotide sequence that, when transcribed and/or expressed, results in the production of an RNA, polypeptide or protein. The nucleotide sequence "encodes" that RNA or it encodes the amino acid sequence for that polypeptide or protein.

The term "polypeptide of interest" means an isolated or synthetic full length protein, an isolated or synthetic full length polypeptide, or an isolated or synthetic full length oligopeptide. The terms polypeptide of interest or protein of interest may be used interchangeably. A protein, polypeptide or oligopeptide has a minimum size of two amino acids. Examples of recombinant polypeptides that can be used in the present invention include polypeptides derived from prokaryotic and eukaryotic organisms. Such organisms include phages, viruses, bacteria, fungi, plant or animals. Types of polypeptides that can be utilized in the present invention include, without limitations, enzymes, structural or membrane proteins, transport proteins, antibodies and other molecules of the immune system, hormones, and messenger proteins. The polypeptide of interest can recognize or bind or otherwise interact with a molecule of interest. The polypeptide of interest may be expressed as part of an expression cassette or by itself. The coding sequence can be a native coding sequence for the polypeptide of interest or may be a coding sequence that has been selected, improved, or optimized for use in the host cell. The polypeptide of interest may be obtained from an antibody library. The polypeptide of interest may be obtained from a single chain antibody library, Fab library, sdAb library, knottin, ankyrin or other scaffold library. The polypeptide of interest may be a single domain antibody. In an embodiment, the polypeptide of interest may begin with a signal sequence. Examples include, without limitations, leader sequences like pe1B or DsbA. In an embodiment, the protein of interest may have a post-translational modification site. Post-translational modifications involve the addition of another moiety to an amino acid molecule of the polypeptide chain and include without limitations biotinylation, glycosylation or ubiquitination. For example, the post-translational modification site may be a biotin substrate site. This site allows a biotinylating agent to add a biotin or a biotin analog to the polypeptide of interest at the biotin substrate site. In one embodiment, the biotin acceptor peptide contains a lysine that serves as the site of biotinylation by *E. coli* biotin ligase though other naturally occurring or artificial biotin ligases may require different substrates. The post-translational modification may also function as a tag. During rapid affinity agent pairing, a polypeptide of interest may function both as a captor and as a tracer.

A "biotin substrate site" means an amino acid sequence to which a biotin or a biotin analog can be added. The biotin substrate site may be a biotin acceptor peptide. The biotin substrate site may be fused to the protein of interest at any position. The biotin substrate site may be C- or N-terminally fused in-frame to the protein of interest. The biotin substrate site may be fused to the protein of interest at an internal position (e.g., a flexible internal loop). The biotin substrate site may be designed to interfere minimally with the function or structure of the protein. The biotinylation of the protein of interest may be performed in a cell free environment or it may be performed in the context of a cell. Once biotinylated, the biotin molecule may also function as a tag for the detection, selection or purification of the polypeptide of interest.

A "biotinylating agent" or an "agent capable of biotinylating the polypeptide of interest" means a chemical or biological entity that can facilitate the attachment of a biotin or a biotin analog at a biotin substrate site. A biotinylating agent may be a biotin ligase. The biotinylating agent may be expressed by a cell, either through an expression vector or may be stably integrated into the cell's genetic framework, or even supplemented into an in vitro reaction as protein, or incorporated synthetically during chemical synthesis of the affinity agent. The biotinylating agent may be part of the expression cassette, carrying the protein of interest. The cell may be a eukaryotic cell or a prokaryotic cell. Examples of eukaryotic cells include but are not limited to a mammalian cell, a *Drosophila* cell, a *Xenopus* cell, a yeast cell or a *C*. *elegans* cell. Providing an agent capable of biotinylating the polypeptide of interest includes all *in vivo* and *in vitro* means of facilitating the attachment of a biotin or a biotin analog at a biotin substrate site. The biotinylating agent may be used with a protein of interest in a cell free environment. For example, without limitation, is biotin protein ligase (birA- Biotin holoenzyme synthetase) that biotinylates the protein of interest at a biotin substrate site, like Biotin Acceptor Peptide. Deletions and low expression level formats of birA like GTG Δ birA may be better suited for the phage display where the acceptor peptide is fused to a phage coat protein. A less modified birA using ATG initiation may be employed more effectively in the trans-display system, resulting in a better subsequent performance in the rapid pairing system. A biotinylating agent is an example of a modification enabling protein. Other modification enabling proteins can be devised to add post-translational modifications to the polypeptide of interest.

A "linker" means a polynucleotide or polypeptide sequence that is used in the expression cassette or fusion protein. A linker may function to introduce cloning sites into the nucleotide sequence. A linker may serve to as a tag for specific molecular interaction, like protein-protein interactions. A linker may provide a flexible component or space-creating region between two protein domains. A linker, separating the biotin substrate site and the polypeptide of interest, may facilitate the correct folding of the polypeptide of interest. Length of the linker is variable. For example, and without limitation, a linker can be a 15-nucleotide sequence coding for the Gly-Gly-Gly-Gly-Ser sequence, which appears to improve the display of the affinity agent and also increases its subsequent production should purification be required.

A "display protein" means an amino acid sequence or a peptide or a protein that directs the presentation of a protein of interest. A display protein may be a surface protein. Examples of surfaces for displaying the protein of interest may be, without limitation, the periplasm, outer cell membranes, inner cell membranes, or phage coat. A display protein may be a secretory protein, whereby the polypeptide of interest is now secreted by the host cell. The display protein may be encoded by the same nucleic acid composition as the polypeptide of interest. The display protein may be encoded on a different nucleic acid composition, or in the chromosome of the host cell. The display protein may be encoded in frame with an affinity agent. A two nucleic acid construct system may be devised, where one nucleic acid construct encodes a polypeptide of interest with a post-translational modification site. And the second nucleic acid construct encodes a display protein and an affinity agent for that posttranslational modification. Interaction between the post-translational modification and the affinity agent leads to the sequestering of the polypeptide of interest and the display protein. The entire complex is presented by the display protein on its destined surface. By changing the nature of the display protein, one can switch from bacterial surface display to phage surface display, without altering the construct that contains the polypeptide of interest. Various surface proteins of a phage include the minor coat proteins g7p, g9p, g3p and g6p, and the major coat protein g8p. Various surface proteins of *E. coli* include ice nucleation protein, ompA, and PAL. Inner surface proteins anchor the polypeptide to the inner side of the outer membrane to face the periplasm, the periplasmic side of the inner membrane (e.g. APEX) or the inner side of the inner membrane. In a cell-free system, the display protein may be associated with a ribosome or a mRNA or dsDNA display.

A "termination codon" or a stop codon means a nucleic acid sequence that signals the protein translation machinery in the cell to stop further translation of the sequence. A termination codon may be one of the three natural codons-UAA, UAG or UGA. A termination codon may also be synthetic translation termination sequence. When associated with a suppressor tRNA that recognizes the stop codon, the cell's translation machinery reads through the stop codon and produces the protein coded by the sequence subsequent to the termination codon. In an embodiment, a vector with a termination codon separating the polypeptide of choice and the display protein may be co-expressed with a suppressor tRNA. The termination codon facilitates the switch in expression of the polypeptide of interest from a soluble form to a surface-displayed protein. In another embodiment, a termination codon may be present between the affinity agent and a display protein. This facilitates the production of independent affinity agents and also affinity agents fused to the display protein. When utilizing any termination codon as this molecular switch, it would be beneficial to ensure that the termination of transcription of the subsequent proteins in the expression cassette is not affected. When a tRNA is said to recognize a codon, it refers to the process by which the anticodon of tRNA binds to the codon of the mRNA and delivers an appropriate amino acid to the ribosome for further polypeptide synthesis.

The term "tag" means a chemical or biological entity, which facilitates isolation, purification or detection of a polypeptide containing the entity. For example, without limitation, a tag may be an amino acid sequence, or a nucleotide sequence that encodes an amino acid sequence. A wide variety of such tags are known to those skilled in the art, and are suitable for use in the present invention. Suitable tags include, but are not limited to, HA peptide, polyhistidine peptides, streptavidin, and other antibody epitope binding sites. Tags may be expressed as part of the polypeptide of interest as a fusion protein. Tags may be post-translational modifications, like biotin, that are added after the protein is expressed. A tag may be a chemical entity. For example, without limitations, a tag may be a fluorescent or a radioactive label that is attached to the polypeptide.

"Detecting" a polypeptide of interest means direct or indirect measurement of the interaction between the molecule of interest and the protein of interest. The measurement can be accomplished by various methods known to one skilled in the art and includes, without limitation, absorbance, transmission, mass measurement, fluorescence intensity, spectrometry, luminescence intensity, or by means of a photographic film detection system, an enzymatic-reaction based detection system, or an optical detection system. In an embodiment, the interaction between the molecule of interest and the protein of interest is directly detected. In another embodiment, the interaction between the molecule of interest and the protein of interest is detected by detecting the tag. In another embodiment, the interaction between the molecule of interest and the protein of interest is detected by measuring the interaction of another affinity agent to the molecule of interest, or the protein of interest, or the complex between the molecule of interest and the protein of interest.

An "affinity agent" means a chemical or biological agent that interacts specifically with the molecule of interest, or the polypeptide of interest, or the tag on the polypeptide of interest. Non-limiting examples of affinity agents include aptamers, haptens, peptides, antibodies, antigens, streptavidin, or neutravidin. Affinity agents may be synthetic equivalents of proteins or nucleic acids. The interaction between an affinity agent and its target can include recognition, or binding, or alteration of physical or kinetic properties, or regulation of activity or location. An affinity agent may be a polypeptide that recognizes and binds to a post-translational modification on the polypeptide of interest. An affinity agent may be a biotin affinity agent. Biotin affinity agents may include, without limitations, streptavidin, neutravidin or avidin proteins.

The term "molecule of interest" means any chemical or biological entity of interest for which an affinity agent is desired. A molecule of interest can be without limitation a small molecule, peptide, hormone, antigen, antibody, deoxyribonucleic acid (DNA), ribonucleic acid (RNA), metabolites of the aforementioned materials and other substances of either natural or synthetic origin.

"Binding affinity" means a selective or specific interaction between the molecule of interest and the affinity agent. Binding affinity may include formation of a bond between the molecule of interest and its affinity agent. Binding affinity may also include noncovalent interactions such as hydrophobic bonds, salt links, hydrogen bonds and van der Waals forces. For example, without limitation, an antigen and its specific antibody exhibit a specific binding affinity.

A "host cell" or "cell" means any cell of any organism that is selected, modified, transformed, grown or used or manipulated in any way for the production of a substance by the cell. For example, a host cell may be one that is manipulated to express a particular gene, a DNA or RNA sequence, a protein or an enzyme. Host cells can further be used for screening or other assays to detect the presence of the particular biological product. Host cells may be cultured in vitro or as one or more cells in a non-human animal (e.g., a transgenic animal or a transiently transfected animal). Nucleic acid compositions according to the invention can be introduced into the target cells by various methods known to one skilled in the art. In an embodiment, the nucleic acid compositions can be expressed in cell-free systems. In another embodiment, the nucleic acid compositions can be expressed in synthetic formulations that support transcription and translation, for example liposomal formulations or encapsulated aqueous compartments.

An "antibody library" refers to a plurality of DNA or RNA molecules containing an open reading frame that encodes an antibody or fragment therof. It also includes a plurality of polypeptides expressed from said DNA or RNA molecules. An antibody library may be available for further screening for specific properties.

A "single domain antibody" refers to a small functioning binding unit of an antibody. A single domain antibody usually corresponds to the variable region of heavy chain only antibodies, though may refer to suitably engineered (e.g. "camelized") heavy variable or light variable regions of conventional (heavy+light chain) antibodies, or variable regions exhibiting favorable solubility

A "suppressor tRNA" means a transfer RNA molecule that causes the incorporation of an amino acid in a polypeptide in a position corresponding to the termination codon in the mRNA being translated. When the stop codon is located at the carboxy terminus of a nucleotide sequence, then the expression of a product with a "native" carboxy end amino acid sequence occurs under non-suppressing conditions (i.e., when the suppressor tRNA is not expressed). While under suppressing conditions (i.e., when the suppressor tRNA is expressed), the protein translation machinery continues to translate the sequence subsequent to the termination codon, resulting in a fusion protein. Any tRNA suppressor, natural, synthetic or otherwise, and its matched stop codon pair may be used in the practice of the present invention. For example if an amber codon is used to separate the protein of interest from a display protein, then the expression of tRNA supE will switch a portion of the expression of the protein of interest from a soluble form to display form. Point mutations like V88 and V89 may increase the suppression. The gene for the suppressor tRNA may be in the genome of the host cell. The gene for the tRNA suppressor may be located on the same nucleic acid composition or on a separate nucleic acid composition than the protein of interest. The vector containing the suppressor tRNA gene may have an origin of replication selected so as to be compatible with the vector containing the expression cassette. The genes may be expressed from the same or different promoters as compared to the promoter used to express the nucleic acid encoding the protein of interest. Two or more different suppressor tRNA-termination codon pairs may be provided.

An "array" means an ordered arrangement of immobilized molecules on a substrate. For example, a substrate may, without limitations, take the form of a particle, bead, gel, matrix, membrane, filter, chip, well, flowcell or a trench. The substrate may be coated with an affinity agent, or a polypeptide of interest or any other molecule of interest. An array may contain a plurality of immobilized molecules. An array may contain a plurality of arrangements of the same molecule but separated from one another, such that separate samples can contact each arrangement.

The present inventions will be described more fully hereinafter with reference to the accompanying drawings in which embodiments of the invention are shown. These inventions may, however, be embodied in many different forms and should not be construed as limited to the exemplary embodiments set forth herein; rather, these embodiments are provided so that this disclosure will be thorough and complete, and will fully convey the scope of the inventions to those skilled in the art.

### Generation of biotinylated proteins of interest.

Nucleic acid compositions encoding polypeptides of interest are designed and formulated to obtain the desired level of transfer, replication and expression efficiency of the polypeptide of interest inside host cells. These nucleic acid compositions may be designed to function in cell-free expression systems or other *in vitro* systems. Generally, nucleic acid compositions are prepared to include a promoter sequence, and genes coding a selectable marker, a protein of interest, and a tag. A post-translational modification site may be cloned in-frame with the gene for the protein of interest, and this may also function as a tag. Standard recombinant DNA methods can be used to obtain desired nucleic acid compositions. Cells can be prepared by starting with competent cells and introducing the exogenous nucleic acid compositions. Methods of introducing exogenous nucleic acids are known to one skilled in the art and include transformation, transfection, transduction or other physical or chemical or biological means. Cells containing the desired nucleic acid compositions are selectively propagated by using markers like antibiotic resistance or color expression systems. Host cells, containing the expression vector for the protein of interest, are generally selected and grown in appropriate media to desired cell population levels. Cells are said to be incubated when they are grown or maintained in the desired media at desired temperature, humidity, CO₂, O₂ and other conditions. When cells are incubated under "conditions sufficient for protein expression", the cells have all the nutrients, growth factors, and other materials and reagents required for expressing the polypeptides encoded by the nucleic acid compositions, such as the polypeptides of interest or affinity agents. When cells are incubated in the presence of an inducing agent in the media, then the inducing agent acts upon the promoter present on the nucleic acid compositions inside the cell. This drives the expression of the downstream expression cassette and thus producing the proteins encoded by the expression cassette. For example, and without limitation, IPTG (isopropyl-beta-D-thiogalactopyranoside) induces the expression of proteins that are under the control of the lac promoter. The protein of interest, thus produced, may be identified, modified or purified by taking advantage of the distinct immunological, enzymatic or physical properties of the polypeptide. For example, and without limitation, if a protein or polypeptide has a unique enzymatic activity, an assay for that activity can be performed on the culture medium used by the host cells. Antibodies that recognize the polypeptide can be used to detect the protein or polypeptide in any immunological assay.

"Cellular fraction" means a collection of cells, including host cells which contain the polypeptide of interest, wherein such cells have been removed from their growth environment. Cellular fraction for example, without limitations, include cell lysates, where the cell membranes are disrupted by physiological or chemical means, and cell shockates. Cellular fractions may include semi-purified or purified fractions of cellular extracts.

Chemical agents or biological agents may catalyze the post-translational modifications of the polypeptide of interest and this may happen *in vivo* or *in vitro.* For example, and without limitation, a post-translational modification like biotinylation may occur in a cell-free system, wherein an exogenous biotin ligase facilitates the addition of a biotin molecule at the biotin acceptor site of a polypeptide of interest. This *in vitro* biotinylation involves exposing cellular fractions containing the polypeptide of interest with the biotin substrate site to the biotin ligase reaction mixture. In an *in vivo* embodiment, a biotin ligase may be encoded as part of the same nucleic acid composition as the polypeptide of interest. When expressed from the expression cassette after induction, this biotin ligase can add a biotin molecule at the biotin acceptor site of a polypeptide of interest. A biotin ligase may be encoded by a different nucleic acid composition than the polypeptide of interest. Exogenous biotin may be provided to the host cells. Sufficient quantity of biotin may be present in the cell culture media to enable successful display and periplasmic production.

A host cell may contain several nucleic acid compositions. In an embodiment, the first vector comprises a first promoter and an expression cassette encoding a polypeptide of interest and a downstream display protein. The gene sequences for the polypeptide of interest and the display protein are separated by a termination codon. And the second vector in the same cell contains a second promoter and a suppressor tRNA gene that recognizes the termination codon in the first vector and suppresses the termination of protein synthesis. When the only inducing agent in the media is against the first promoter, the translation machinery of the cell stops at the termination codon and produces only the polypeptide of interest. This polypeptide is present in a soluble form. When there are inducing agents for both promoters, the expression of the suppressor tRNA leads to the production of a proportion of the polypeptide of interest attached to the display protein. Depending on the nature of the display protein, the polypeptide of interest may now be presented on a cellular or viral surface (or may be on ribosome if using an *in vitro* reaction). If the display protein contains the sequence for the phage coat, then the polypeptide of interest is presented on the phage coat. If the display protein moves to the periplasmic space, then the polypeptide of interest is presented in the periplasmic space. Thus by altering the presence of the inducing agents, one can change the expression profile of the polypeptide of interest from a soluble form to a displayed form. For example, without limitation, using inducible supE expression systems enabled single domain antibody populations to be propagated as phage for more panning from repertoires or expressed as soluble antibodies for screening within a single host strain. The supE expression system provides for the expression of a mutant tRNA that will suppress the reading of the termination codon and permit the addition of an amino acid to the growing polypeptide chain.

In another embodiment, the host cell comprises two nucleic acid compositions. The first vector comprises a first promoter and an expression cassette encoding a polypeptide of interest and a post-translational modification site. The second comprises a second promoter, and a second expression cassette encoding a downstream display protein and an affinity agent for the post-translational modification. For example, and without limitation, the post-translational modification site may be a biotin acceptor peptide and the corresponding affinity agent may be streptavidin. The interaction between the post-translational modification and the affinity agent leads to sequestering of the polypeptide of interest by the display protein. So by altering the display protein, the polypeptide of interest can be directed to any cellular surface of choice without re-engineering the polypeptide of interest into other vector constructs. The nucleic acid composition comprising the polypeptide of interest may be used with display systems in different host cells. For example, by using yeast display proteins in a yeast host cell, the polypeptide of choice can be displayed on yeast cell surfaces. For example, without limitations, an inducible supE expression system was combined with the expression of streptavidin as a fusion protein with the gene 3 protein (g3p) of M13 bacteriophage. This enabled a semi-synthetic single domain antibody library, which was mined for anti-*Ebola* virus antibodies, to be readily available for immunoassays without requiring recombinant protein purification.

There are advantages to the transdisplay system, where the display protein with the affinity agent and the polypeptide of interest with the affinity tag are on separate nucleic acid compositions. The nucleic acid composition, in which the library of polypeptides of interest is assembled, lacks the gene for the display protein and is therefore more efficiently transfected into host cells enabling larger repertoires to be assembled. For example, and without limitation, when an existing single domain antibody library was adapted to either the rapid ligand pairing system (pecan126) and the transdisplay system (pecan133/134 similarly armed), the 133 library size was approximately ten-fold larger. As the transcription of the polypeptide of interest and the display protein may be uncoupled, a stronger promoter may be utilized for the expression of the polypeptide of interest. This will help avoid the toxicity or immunity to superinfection caused by overexpression of the display protein. Using a strong promoter for the polypeptide of interest will enable facile characterization and subsequent purification if required. Certain polypeptides of interest may have C-terminal folding challenges. For example, and without limitation, a protein can have a buried C-terminal structure, so displaying such polypeptides as direct fusion products with the display protein may not be feasible. Transdisplay systems may alleviate this as the polypeptides of interest may be produced with only a tag and/or a flexible linker. In another embodiment, a multimeric affinity agent may be devised such that it binds to more than one polypeptide of interest with the affinity tag. Thus, low affinity clones may be selected by virtue of avidity. Different polypeptides of interest may be displayed simultaneously, by taking advantage of the multimeric nature of the affinity agent.

### Rapid affinity agent pairing.

Mining protein libraries for specific properties against the molecule of interest is a powerful technique in molecular biology. It has applications in diagnostics, prognostics, therapeutics and other uses that take advantage of protein-molecule of interest interactions. The invention provides for screening methods for identifying nucleic acid molecules, bacteriophages that may contain them, or the host cells that encode the polypeptides of interest against a molecule of interest. The molecule of interest may be a chemical or a biological entity. The molecule of interest may be a nucleic-acid-based or amino acid-based composition, or may be a naturally occurring or synthetic small molecule. For example, and without limitations, the molecule of interest may be a viral protein or a bacterial surface protein. A polypeptide of interest may be a receptor with specific binding to a ligand of interest. A polypeptide of interest may be an antibody that binds specifically to an antigen of interest. In an embodiment, host cells may contain polypeptides of interest that constitute one or more libraries and may encode variable domains of antibody light and heavy chains.

In an embodiment, a library of clones displayed on phage is screened for specific properties against a molecule of interest. Specific properties may include greater binding affinities or greater stability. Clones, that test positive for the desired characteristics, are mobilized to an expression host, and expressed as discussed supra. In one embodiment, single domain antibody clones were developed against a particular molecule of interest. In order to develop diagnostic reagents, two non-competing polypeptides of interest have to be identified - one polypeptide to bind to the molecule of interest and another to recognize that bound polypeptide complex. The two polypeptides have to be unique in some fashion to allow discrimination between the two in the signal amplification step. Developing and utilizing two reagents is both time and cost inefficient. This inefficiency arises due to the demand for protein purification and modification to distinguish the captor from the tracer. For example, this pairing creates a bottle-neck in recombinant antibody sandwich immunoassay development. One embodiment of the present invention is a method of utilizing the same polypeptide of interest, without any further modification, as both a captor and a tracer. For example, without limitations, an expression system was developed with biotinylated captor antibodies. A single biotin associated captor antibody was bound by a neutravidin coated platform, and the same style of biotinylated antibody was used as a tracer. The subsequent neutravidin-enzyme secondary conjugate only recognized the free tracer biotin and not the occluded biotin on the captor. Both avidin and streptavidin have high affinities for biotin. This combined with the almost quantitative concealment of the bound hapten ensured an almost irreversible captor platform with no background binding. This system provided independence from DNA sequencing or antibody purification. This system may be used in any environment but has particular use in high containment laboratories since no agents need to be removed from the laboratory to develop diagnostics and therapeutic antibodies against highly infectious pathogens. For example, without limitations, a simple pairing system was developed using microliter amounts of *E. coli* osmotic shockates containing site-specific biotinylated antibodies. These single domain antibodies performed as both the captor and tracer for polyvalent *Marburg* virus nucleoprotein. As another example, pairs of single domain antibodies were developed to recognize seven botulinum (BoNT) serotypes, enabling specific recognition of the cognate serotype.

In one embodiment, the array is designed to present affinity agents for the tag present on the polypeptide of interest, The tag may be a post-translational modification on the protein, or another tag expressed in frame with the polypeptide of interest. The polypeptide of interest may be a purified preparation or a semi-purified preparation. The polypeptide of interest may be present in a purified or unpurified or semi-purified cellular fraction. This strategy permits the use of crude cellular fractions, without several rounds of purification. Cellular fractions include, without limitations, whole cell lysate, cell shockate, osmotic shockate or semi-purified fractions thereof. The polypeptide of interest that binds to its affinity agent on an array functions as the captor. An array with the affinity agent bound to the captor is treated with the molecule of interest that is recognized by the captor. Then the cellular fraction containing the polypeptide of interest is again added and it now performs as the tracer. The captor-bound molecule of interest binds to the tracer and this entire complex may be detected by using the tag on the tracer. The tag may be detected using a detection system such as but not limited to fluorescent detection system, a luminescent detection system, a photographic film detection system, an enzyme detection system, or an optical detection system.

For example, and without limitation, an array may be devised with biotin affinity agents, like neutravidin. When the cellular fraction with the biotinylated polypeptides of interest is added, the neutravidin selectively binds the biotinylated polypeptides. The molecule of interest is subsequently allowed to bind to the bound polypeptides. Then the same preparation of biotinylated polypeptides used in the previous step is added to the bound molecule of interest. The biotinylated polypeptides of interest now function as the tracer. The biotin attached to the tracer polypeptides facilitates the detection of the bound complex.

The method may include saturation of the binding sites on the affinity agent after the first binding with the biotinylated polypeptides. Saturation may be achieved by blocking agents that contain biotin such as non-fat dried milk. In one embodiment, the method further comprises removing unbound biotinylated proteins prior to detecting bound biotinylated proteins. The method may include other steps known in the art to facilitate binding interactions, like washing between the various steps to eliminate non-specific binding.

While these embodiments have been described with emphasis on the embodiments, it should be understood that within the scope of the appended claims, the embodiments might be practiced other than as specifically described herein. Although the invention has been shown in only a few of its forms, it should be apparent to those skilled in the art that it is not so limited but susceptible to various changes without departing from the scope of the invention. Accordingly, it is intended to embrace all such alternatives, modifications, and variations as fall within the spirit and broad scope of the appended claims.

Those skilled in the art will recognize that many changes and modifications may be made to the method of practicing the invention without departing the scope and spirit of the invention. In the drawings and specification, there have been disclosed embodiments of the invention and, although specific terms are employed, they are used in a generic and descriptive sense only and not for the purpose of limitation, the scope of the invention being set forth in the following claims. The invention has been described in considerable detail with specific reference to these illustrated embodiments. It will be apparent, however, that various modifications and changes can be made within the spirit and scope of the invention as described in the foregoing specification. Furthermore, language referring to order, such as first and second, should be understood in an exemplary sense and not in a limiting sense. For example, those skilled in the art may recognize that certain steps can be combined into a single step.

### EXAMPLES

The following examples further illustrate the compositions and methods.

### Example 1

### Example 1A

An example of the molecular basis of the affinity agent pairing system is illustrated in **FIG. 1A**. An affinity agent like neutravidin (cross) is passively absorbed on a surface (black line). Crude osmotic shockate is applied to the system. This shockate contains the protein of interest, like a biotinylated single domain antibody (pointed tab). The biotin moiety (pointed end of tab) facilitates the binding of the antibody to the neutravidin. This shockate functions as the source of both antigen capture and antigen tracing, with distinction by the secondary enzyme conjugate defined as the ratio of signal to noise. Unoccupied biotin binding sites on the neutravidin can be blocked with biotin (triangle) in milk. The molecule of interest, like an antigen (branch) is added and is captured by the immobilized neutravidin-sdAb combination. Crude osmotic shockate is again applied and the antigen captures more sdAbs. Now the biontinylated sdAbs function as the tracer. Neutravidin charged with horseradish peroxidase (cross with lightning bolts) is added which can only bind to that sdAb that has free biotin. The bound complex is detected through horseradish peroxidase detection system. This system produces a colored, fluorometric, or luminescent derivative of the labeled molecule when incubated with a proper substrate, allowing it to be detected and quantified.

### Example 1B

**FIG. 1B** shows the initial expression constructs that were engineered to generate antibodies to serve as examples of affinity agents compatible with the rapid pairing system. These construct designs were used to balance *in vivo* sdAb biotinylation with phage display compatibility and effectiveness of capturing/tracing. Pecan is a vector designation and basal vectors used to establish the rapid pairing system are all based on pecan114. This vector pecan114 is a tac promoter-based high copy number vector driving expression of a His6 tagged sdAb to an amber codon and through to M13K07g3p (g3p) in the appropriate strain. The BAP was inserted between the sdAb and His6 tag with flexible Gly4Ser linkers (G4S-BAP-G4S) to yield pecan122. The *birA* from *E. coli* DH10B chromosome was inserted with a ribosome binding site downstream of g3p to provide extra biotinylating capacity in pecan123. Since overexpression of birA maybe deleterious to phage display, constructs with various mutations were developed, like constructs with poorly initiated birA (pecan124), the deletion of the DNA binding motif (pecan125) and a poorly initiating version (pecan126).

### Example 1C

Trials of vectors from Example 1B in the rapid pairing system revealed that all vectors containing the BAP were functional when crude osmotic shockate was used as captor and matching phage was used as tracer (**FIG. 1C**). Titrating crude osmotic shockates from HB2151 cultures were used as captors and 10 µL of supernatant from M13K07 infected XL1-Blue cultures were used as tracers. The requirement of the biotin acceptor peptide (BAP) sequence for sdAb capture was demonstrated (compare pecan114 and 126 in **FIG. 1C**). Superior signals were obtained when a biotin ligase (birA) cistron was included in addition to the BAP (pecan123-126) especially when the birA DNA binding domain was absent and a poor translation initiation signal was employed (pecan 126).

### Example 1D

When crude shockate was used as tracer, there was an almost absolute requirement for a second cistron of birA (**FIG. 1D**). Using a constant 1 µL of the shockate as captor, and titrating the same shockate as tracer reveal that the sdAb pairing system works well though with a 10-100 fold drop in sensitivity from phage tracer, and shows the need for the second cistron birA in the face of this signal decrease (**FIG. 1D**). The pecan122 construct yields very low signals compared to pecan114 despite having the BAP sequence. Overexpression of birA in pecan123 appears to work well, with both shorter birA being less effective, especially when poorly initiated. Note the complete absence of background ELISA signal when using negative control antigen Ebolavirus Zaire nucleoprotein, indicating the pairing system is very clean, with secondary conjugate not appearing to detect rogue immobilized captor sdAb or background binding tracer sdAb.

The difference in behavior between the two assays can be rationalized by each bound phage is recognized by hundreds of copies of the anti-phage g8p HRP and amplifying any positive signal, while each bound sdAb will be recognized only by a single neutravidin HRP.

### Example 1E

**FIG. 1E** demonstrates that the antigens used are specifically capable of polyvalent binding with conventionally immobilized non-biotinylated sdAb and sdAb-alkaline phosphatase tracers. Recombinant Marburg and Ebola Zaire virus nucleoprotein mimics are polyvalent within crude cytosolic extracts of *E. coli* and therefore are acceptable surrogates for finding the affinity agents at biosafety level 2 (BSL-2). Representatives of unique sdAb genes were mobilized to pecan22, a high level periplasmic expression vector based on pMoPac10 but encoding only a C-terminal His6 tag instead of His-myc tag. Host *E. coli* Tuner™ strains with pRARE bearing tac promoter-based pecan42 driving cytosolic expression of tagless Marburg virus Musoke (MBG) or Ebolavirus Zaire Kikwit (EBO) nucleoprotein genes were generated. Clarified lysates of these *E. coli* were titrated over duplicate wells coated with 100 µL of 100 nM unbiotinylated sdAb derived from the pecan22. EBOZ C was a clone selected on the Zaire virus and MBG C was a clone selected on the Marburg virus using standard display methods. Antigen capture was detected with 100 µL of 100 nM sdAb-AP fusion and Pico-West chemiluminescent detection.

### Example 1F and 1G

A series of experiments were conducted to examine the effect of different biotin ligase mutations in the generation of the rapid affinity agent system. All vectors described in Example 2 yielded equivalent quantities and purities of sdAb following mid-scale expression (400 mL shake-flask culture), and purification by immobilized metal ion affinity chromatography (IMAC) and gel filtration. The propensity to produce phage displayed sdAb, normalized for binding to directly immobilized antigen was examined among constructs having different biotin ligase mutations in two different host cells. XL-1Blue is a constitutive supE positive host, while HBV88 is an inducible host working in the display mode (**FIGS. 1F** **and** **1G**). Since the starting source for the rapid pairing method may be immune or non-immune libraries, the vector producing high antigen binding responses like pecan 126 may be desired Though full-length ATG-initiated birA (pecan123) may have a mild advantage when using sdAb-sdAb pairing, the drop in signal when using it for sdAb-phage pairing indicated potential incompatibility with effective phage display (confirmed in **FIGS. 1F** **and** **1G**), thus only the GTGΔbirA platform was pursued further.

### Example 1H and 1I

A series of experiments were conducted to examine the effect of variations in linkers between the BAP sequence and the sdAb/ His6 tag in the generation of the rapid affinity agent system. Pecan is the vector designation and this experiment involves pecan114 having no BAP, and pecan126-132 having BAP tags but with varying linker regions. The pecan126 hasG4S linkers on both sides of the BAP. The pecan130 has no G4S linkers on either side of the BAP. The pecan131 has the G4S only at the 5'end of the BAP. The pecan 132 has the G4S only at the 3'end of the BAP. These constructs were examined in two different host cells - (**FIGS.1H** **and** **1I**).

### Example 1J

While establishing the pairing system, a novel host strain was engineered that enables both low level expression of platform genes for phage display and high level expression for soluble sdAb production. The strain has advantageous as shown below. A schematic of the system is shown in **FIG.1J** and relies upon the provision of an inducible synthetic suppressor tRNA that conditionally reads through the amber codon of the display vectors.

In the schematic drawing in **FIG. 1J****,** the interactions in the host cell that lead to the phage display mode of the polypeptide of interest are shown above the dashed line. Arabinose induction allows suppression of low level sdAb-amber-g3p translation and subsequent sdAb display. In soluble expression mode (below the dashed line), the amber codon is not suppressed and full induction with IPTG generates large amounts of periplasmic sdAb. The three vectors - KO7 replicative form, pSCUPERV88 and pecan126 - are represented as three large bold circles, and the supE V88 tRNA as a clover leaf. The sdAb are represented as small circles and the g3p are represented as ovoids. Since pecan is a phagemid, it is preferentially packaged during display over the KO7 DNA. The host strain of HB2151 + pSCUPERV88 is known as HBV88 and is used in this example. Consequently, HBV88 is highly effective at being able to express soluble sdAb when the supE V88 is switched off, and highly effective at being able to display sdAb on helper phage when the supE V88 is switched on (**FIG. 1J**). HBV88 is capable of producing equivalent quantities and purities of sdAb as HB2151 following mid-scale expression and purification.

### Example 1K

In cells expressing the suppressor tRNA supE, such as TG-1 or XL1-Blue, the amber codon is constitutively partially suppressed and fusions to a coat protein will be produced and assembled into phage if the cell is superinfected with helper phage. In this example, g3p was the coat or display protein used and M13K07 was the helper phage. In cells lacking supE tRNA, no read-through will occur and only soluble sdAb will be produced. Conventionally, phage panning hosts are always supE+ve and so any progeny phage that are positive by monoclonal phage ELISA must be transferred to a non-supE host such as HB2151. The transfer process leads to doubling of stored clones and therefore, more prone to errors/mix-ups. This can hamper concise characterization. Furthermore, the obligation to use a strain for phage display that constitutively expresses supE and hence high levels of g3p fusion protein can result in immunity to superinfection, and precludes the use of strong promoters for effective sdAb production and subsequent pairing. This apparently conflicting situation may be alleviated by engineering a plasmid that is compatible with pecan display vectors and conditionally expresses supE.

The vector pAR3 was initially chosen as a vehicle for supE, which is a p15a origin chloramphenicol-resistant arabinose-inducible plasmid that is compatible with the colE1 origin of the display phagemids. Though M13K07 helper phage also has a p15a origin, the different resistance genes between phage and plasmid should allow both to be selected for within the same cell. A supE tRNA was cloned as a synthetic oligonucleotide bridge within the polylinker of pAR3 (Perez-Perez and Gutierrez, 1995) to yield an arabinose-inducible supE cassette, pAR3supE. Data points for pAR3 are occluded on the graph by the data points for pAR3supE. p15a is quite a low copy number origin, and levels of suppression were insufficient in HB2151 bearing basal display vector pecan114 when compared with XL-1 Blue (constitutively supE positive) (**FIG. 1K**). Therefore, the gene dosage was elevated by fusing the chloramphenicol-resistant arabinose-inducible supE region of pAR3supE with the origin of an elevated copy number variant of pSC101 which enabled low but detectable display. pSC101 is even lower than p15a, yet two high copy number mutants are known (Peterson and Phillips, 2008) with one exceeding p15a and so a chimera between pSC101 repA E→R and the Cm-Ara-supE portion of pAR3-supE was made using the splicing by overlap extension PCR approach to create pSCUPER. The resulting plasmid began to show detectable levels of suppression of the amber codon within pecan114 when both plasmids were maintained in the host HB2151. Increased suppression was afforded by replacing the wild type supE gene with two individual point mutants known to increase suppression known as Su+2-88 and Su+2-89 (nicknamed herein as V88 and V89) (Bradley, *et al.,* 1981). The resulting strain of HB2151+pSCUPER-V88 was denoted HBV88 and was first employed to study variants of the linkers flanking the BAP sequence for impact on display to reveal both linkers appear advantageous (**FIGS. 1H****-1I).** This strain HBV88 can be made electro competent by standard procedures to almost 1e+9 cfu/µg pUC and is therefore suitable for library generation.

### Example 1L

The supE vehicles were compared in a strain other than HB2151 by conditionally transforming non-suppressor hosts into supE positive display hosts. The highly electrocompetent strain DH10B was conjugated with XL1-Blue and progeny selected on streptomycin and tetracycline to make DH10F'tet, a host suitable for phage display trials. DH10F'tet bearing pecan114 and the supE plasmids were superinfected with M13K07 and induced with 10 µM IPTG. Supernatants were analyzed for capture by recombinant Marburgvirus NP (**FIG. 1L**). Control signals on Ebolavirus NP were -0.010 to -0.002. This example demonstrates that the supE vehicles can perform in hosts other than HB2151.

### Example 1M and 1N

Constructs with variations in the linker region, as described in Example 1H and 1I, were expressed in HB2151 and HBV88 host cells. Soluble sdAb production from equivalent wet weights of cells were analyzed by gel filtration UV chromatograms of IMAC purified fractions. The parental pecan126 is a superior producer among the BAP tag vectors if scale up should be required (**FIGS. 1M** **and** **N**). **FIG. 1M-1** shows protein production from constructs - pecan73, pecan114, and pecan126 - in HB2151 strain. **FIG. 1M-2** shows protein production from constructs - pecan130, pecan131, and pecan132 - in HB2151 strain. **FIG. 1N-1** shows protein production from constructs pecan73, pecan114, and pecan126 - in HBV88 strain. **FIG. 1M-2** shows protein production from constructs pecan130, pecan131, and pecan132 - in HBV88 strain. Typical 400 mL shake flask cultures reliably yield approx. 15 mg of highly pure sdAb from pecan126. The yield from pecan126 and 132 is approx.75% of the yield of pecan114 (the basal vector without a BAP sequence), whereas pecan130 and 131 are approx 30%. Furthermore, pecan126 consistently generated at least 30-100% more pure protein than pecan130-132 (**FIGS. 1M** **and** **1N**). The differential production indicates the nature of flexibility around the BAP tag can influence yield.

Although the absence of the trailing Gly4Ser caused an approximate three-fold drop in soluble sdAb expression (**FIGS. 1M** **and** **1N**), all constructs generated highly pure sdAb as judged by Coomassie stained SDS-PAGE (**FIG. 1O**) with mass-spectrometry of the peak fractions revealing unbiotinylated and biotinylated sAb **(****FIG. 1P****).**

### Example 1O

Protein expression from all constructs were analyzed on Coomassie stained SDS-PAGE (**FIG. 1O****).** Major peak fractions from HB2151 and HBV88 mid-scale preps of the linker variant vectors revealed equivalent extents of purification from each of the vectors afforded by standard immobilized metal ion affinity chromatography (IMAC) and gel filtration. (**FIG. 1O**). 8 µL of the peak and flanking fractions were combined with 32 µL of water and 40 µL of Laemmli reducing sample buffer, boiled for 3 min, cooled and 20 µL of each sample was loaded on 15% Laemmli SDS-PAGE. Lane M represents unstained molecular weight markers (Biorad) in kDa. The doublets may be a consequence of BAP secondary structure.

### Example 1P

Mass spectrometric analysis of the central peak fractions of pure sdAb preparations were conducted to discriminate between unbiotinylated and biotinylated protein regardless of secondary structures occurring in the linker variants. The predicted masses of the unbiotinylated and biotinylated proteins are:
13,837 Da for pecan73 (**FIG. 1P-1****)** and pecan114 (**FIG. 1P-2**) - these do not have any biotinylated proteins;
16,278 and 16,278+ 226 Da for pecan126 (**FIG. 1P-3****);**
15,648 and 15,648 + 226 Da for pecan130 (**FIG. 1P**-**4**)**;**
15,963 and 15,963 + 226 Da for pecan131 (**F1G. 1P-5);** and
15,963 and 15,963 + 226 Da for pecan132 (**FIG. 1P-6**).

### Example 1Q and 1R

All constructs within HBV88 enabled sdAb pairing thus confirming the dual utility of the new host strain. (**FIGS. 1Q** **and** **1R**). In the following examples, a sdAb specific for a polyvalent antigen (Marburg virus nucleoprotein) was constructed, expressed as a BAP fusion and extracted from small-scale culture by osmotic shocking. ELISA plates coated with neutravidin were used to absorb varying amounts of the shockate. Plates were then washed with the antigen or the negative control antigen applied in milk. Following washing, the captured antigen was detected by applying varying amounts of the shockate followed by a constant amount of neutravidin HRP. **FIGS. 1Q** **and** **1R** have two graphs that show the single species of sdAb produced within the host cell HBV88 recognizing a polyvalent antigen, demonstrated by the highly specific ELISA signals on positive control surrogate antigen (**FIG. 1Q**) but not negative control surrogate antigen (**FIG. 1R**). As the captor biotin is occluded by the immobilized neutravidin, it is not visible to the tracing neutravidin-HRP as shown by negative signals using the Ebola virus nucleoprotein control (**FIG. 1R**).

### Example 1S

This example shows the use of the present method in identifying pairs of antibodies and their complementary antigens. One vector pecan132, was used within HBV88 to host a panel of sdAb that forms specific pairs capable of recognizing each of the 7 BoNT toxins to confirm specific recognition of the cognate toxin serotype using just crude shockates (**FIG. IS**). The sdAbs were produced in the pecan132 system and isolated as crude osmotic shocks. Crude shockates of HBV88 were used as a source of captor and tracer, with a fixed 1 µg/mL of toxin used. Each pair was used as a captor-tracer combination and challenged with a set concentration of each of the 7 BoNT serotypes to ensure specificity was retained, i.e., pair A bound only BoNTA, pair B bound only BoNT B etc. As shown, specificity is retained on par with earlier findings on Luminex (the anti-D pair was raised against a C/D mosaic and hence shows reactivity with C and has also shown to cross-react with G).

### Example 1T-1V

Further tests were conducted to analyze the ability of the vectors to enable enrichment during a panning process. The heptaplex anti-botulinum neurotoxin library made in pecan21 was subcloned to either pecan126 or pecan133 to evaluate HBV88 and HB+p134 respectively. Library sizes:
- Pecan21 - 1e+9 cfu total size, rescued to 2.2e+11 cfu/mL phagemids, employed 100 µL in pan (22 representations)
- Pecan126 - 3e+8 cfu total size, rescued to 5.4 e+12 cfu/mL phagemids, employed 100 µL in pan (180 representations)
- Pecan133 - 2e+9 cfu total size, rescued to 3e+12 cfu/mL phagemids, employed 100 µL in pan (150 representations)

Amplified eluate phage in suitable strain (pecan21 in XL1 Blue) was plated, scraped and superinfected for rescue overnight. 50 µL of the crude supernatant and an equivalent amount of pre-panned library were analyzed by ELISA on all seven serotypes plus ovalbumin control (coat lug/mL). Positive binding clones were revealed with anti-phage-HRP. All libraries appear to be enriched for BoNT specific clones with pecan126 outperforming the 133/134 system. Both 126 and 133/134 systems have higher background on all other coats including ovalbumin suggesting an inherent stickiness over parental pecan21 (**FIG. 1T**). Both systems successfully enable selection and pairing of clones from a repertoire without the need for strain transfer, subcloning, sequencing or protein expression and purification at scale. Panning was performed by the plate coat method with ten wells coated with 10 µg/mL unbiotinylated BoNT serotype A, blocked with Milk PBS, panned with library, eluted with alkali. **FIG. 1U** is the graph that shows that both the standard display format (pecan21) armed with rapid ligand pairing ability (pecan126) and the transdisplay system (pecan133/134 similarly armed) can generate single domain antibodies specific for their desired target (BoNT serotype A) by screening from libraries of 1e+8 and 1e+9 respectively using a standard panning method. Monoclonal phage ELISA of 96 round 1 clones from HBV88 + pecan126 were screened on 1 µgmL-1 of either BoNT A or control antigen ovalbumin to identify positives for soluble expression and subsequent pairing. **FIG. 1V** is the representation of the same ELISA performed on ovalbumin. Positive clones, like that in well A1 of **FIG. 1U** and **FIG. 1V****,** display a high signal to noise ratio, while negative clones like that in well A5 of **FIG. 1U** and **FIG. 1V****,** display a very low signal to noise ratio. This dataset demonstrates that pecan126 based display can serve as a productive resource for repertoires, enabling them to be rapidly deconvoluted and resulting clones paired.

### Example 1W

Pairing of sdAb clones, identified from pecan126/HBV88 heptaplex BoNT immune library retrofitting, selection and screening, reveals several promising and novel combinations of sdAb capable of recognizing 1 µgmL-1 BoNT serotype A. The twelve highest signal to noise ratios from the monocolonal ELISAs from **FIG. 1U** and **1V** were used in the pairing assays to determine signal to noise ratios in the binding of the sdAbs to the toxin. **FIG. 1W** is the rapid ligand pairing on 1µg/mL BoNT A in solution. Clone B2 is an example of a clone that is highly functional as a captor and a tracer, while clone F1 is an example of a poorly functional clone.

### Example 1X

**FIG. 1X** is the control assay on 1µg/mL ovalbumin. Negative control antigen ovalbumin 1 µg/mL used alongside the experimental pairing reveals no background signals after pairing sdAb clones isolated from the pecan126/HBV88 mediated retrofit of a heptaplex BoNT immune library.

### Example 1Y

Only after pairing was evaluated, sequencing was performed to reveal the 12 clones that were similar to those isolated previously using conventional methods. Predicted amino acid sequences of sdAb clones selected using pecan126 within HBV88 (126) and pecan133/134 within HB1251 (133) were aligned with the original anti-BoNT A clones from conventional panning (**FIG. 1Y**).
The nomenclature for the clones in **FIG. 1Y** is as follows. Clones from the conventional panning method start with the letter A indicating the anti-A serotype. Subsequent letter R and the number following it designate the round of isolation. Alpha-numeric designation following denotes historic ELISA plate well location. The final number designates specific clones, labeled from 1 through 18. Clones selected from Example 1U start with the vector designation 126 followed by the well number from **FIG. 1U****.** Clones selected from Example 21 start with the vector designation 133 followed by the well number from **FIG. 2I****.**

Pecan133 and pecan134 share the lacZ' priming site and must be sequenced with AHX76. Clone D3 from the pecan126 selection could not be fully sequenced, appearing to terminate before CDR3 and clone B8 from the pecan133 selection terminated before the sdAb gene. Sequences were aligned with Multalin.

### Example 2

As another example of the invention, we sought to reduce the size of the display phagemid to increase library sizes via improved transformation efficiencies by moving the platform gene (g3p) to the supE expression plasmid, while still retaining antibody pairing and single host capability. It should be possible for the g3p to display the sdAb in trans via capture of the hapten biotin, if it were afforded a streptavidin (strep) motif as shown in the schematic (**FIG. 2A**) alongside the phagemid-plasmid combination used (**FIG. 2B**). We first tested all of the N-out C-in phage coat proteins for their ability to display a streptavidin minigene as an amber suppressed motif capable of binding biotin. Full-length g3p fusion was the superior display platform (**FIG. 2C**). The strep-coat protein genes were then amplified with a primer encoding supE-V88 and a common back primer. The pSCUPER V88 supE gene was replaced with the new construct and display of streptavidin was verified (**FIG. 2D**). To provide the source of biotinylated sdAb, the g3p region of pecan126 was exchanged for stop codons to create pecan133, and co-transformed into HB2151 with the various supE-V88-streptavidin fusion vectors to reveal that only g3p appeared permissive for trans-display under various IPTG/arabinose combinations (**FIG. 2E**). Finally, the optimal conditions were used in a comparison with XL1-Blue and HBV88 to reveal an approx. ten-fold drop in signal strength by phage monoclonal ELISA (**FIG. 2F**), while pairing showed equivalent signals to the HBV88 system (c.f. **FIG. 2G****/H** with **FIGS. 1Q****/R**) since the soluble expression mechanics are the same between pecan126 and pecan 133. To demonstrate the trans-display system could be useful in repertoire selection the original BoNT library was again retrofitted into pecan133 to make a 2e+9 cfu member library, (twice the size of the original) and used to pan on BoNT serotype A as before to reveal enrichment signal (**FIG. IT**) and monoclonal binders (**FIGS. 2I** **and** **2J**) which were then successfully paired (**FIGS. 2K** **and** **2L**). Again, after the fact DNA sequencing identified similar clones to those isolated previously (**FIG. 1Y**).

### Example 2A

**FIG. 2A** is a schematic of the transdisplay system. The pecan134 vector, a chloramphenicol resistant pSC101 high copy number origin mutant plasmid, expresses supEV88 which suppresses a streptavidin core minigene fused to g3p platform. The pecan133 vector is an ampicillin resistant phagemid that expresses sdAb-BAP and birA only. Induction for soluble sdAb expression is the same as the HBV88 system, but for display the arabinose concentration is 100 fold lower to reduce toxicity from strep-g3p over expression.

The system was also developed to sequester the BAP within a phage displayed streptavidin in trans by modifying the system in **FIG. 1J****.** The displayed ligand (e.g. sdAb) is provided in trans from a pecan like phagemid. Display of the sdAb occurs since core streptavidin-g3p fusion binds to periplasmic sdAb-BAP and carries it out to the supernatant during the course of assembly (**FIG. 2A**). The sdAb with BAP is expressed from pecan133 via IPTG induction and exported to the periplasm to become bound to the core-strep-g3p fusion and subsequently transdisplayed on the assembled phage. As before, a simple alteration of induction conditions enables both phage display and soluble expression to be driven from within the same cell. In terms of display level, the core streptavidin will assemble to a tetramer to be functional and may well capture multiple sdAb up to 4 maximally (each streptavidin tetramer has 4 biotin binding sites). The cell used is HB2151, which has no endogenous supE tRNA.

### Example 2B

**FIG. 2B** is an illustration of the expression constructs that would function in a transdisplay mode and would be compatible with the rapid pairing system. For example, a host cell may have two nucleic acid compositions. The first nucleic acid composition comprises a polypeptide of interest (here it is a single domain antibody) in frame with a biotin substrate site, another affinity tag (e.g. His6) and a biotinylating agent. The second nucleic acid composition comprises genes for a biotin affinity agent (e.g. streptavidin) and a display protein (e.g. g3p). In one embodiment, the genes for the biotin affinity agent and the display protein may be separated by a termination codon (marked as an asterisk on the second construct in **FIG. 2B**). The gene for the suppressor tRNA that recognizes the termination codon may be present as part of the second nucleic acid composition (**FIG. 2B**) or it may be present independently on another nucleic acid composition. In another embodiment, there may be increased expression of the free biotin affinity agent, compared to the expression of the biotin affinity agent fused to the display protein. For example, the termination codon between the streptavidin gene and the display protein gene could be designed such that it permits read-through only about 20% of the time. This would ensure that at least three free streptavidin molecules are present to interact with one fused streptavidin-display protein molecule. This strategy may also eliminate the phage extrusion problems associated with self-assembly of the g3p protein. In another embodiment, the second nucleic acid composition in the host cell may be a dicistronic construct with at least two copies of the biotin affinity agent and no engineered termination codon between the biotin affinity agent and the display protein as there will be no requirement for a suppressor tRNA gene to facilitate the termination codon read-through, there will no rescue of host cells that contain termination codon errors in the polypeptide of interest. This strategy may eliminate the need to repair error-prone PCR libraries, before using the constructs for high-level soluble expression. The pecan133 vector here is driven by the IPTG inducible tac promoter and pecan134 by an arabinose promoter with the amber codon (*).

### Example 2C

ELISA capture of sdAb (sdAb) or core streptavidin (STREP) displayed on the various coat proteins of M13 within a pecan114 backbone in XL-1 Blue were analyzed to choose the platform to proceed for transdisplay. The coating antigen was either Marburg NP protein or an equivalent amount of the purified sdAb-BAP generated from pecan 126 (**FIG. 1O**) to show that all platforms appeared to display enough streptavidin to be specifically bound by biotinylated sdAb.

### Example 2D

Constructs used in Example 2C were used to generate the transdisplay vectors. ELISA capture of pSCUPER backbone vectors having the supE exchanged for supE-streptavidin-platform gene fusions within XL-1 Blue initially was performed to verify that the streptavidin is still sufficiently displayed. All but g9p were capable of display (**FIG. 2D**).

### Example 2E

Single wells from a checker-board optimization of arabinose (2000 to 2 µgmL-1) and IPTG concentrations (1000 to 1 µM) for transdisplay in HB2151 bearing supEV88-streptavidin platforms with pecan133 were analyzed. Only the g3p vector had detectable signals at any combination of arabinose and IPTG (see **FIG. 2E** **-** high value for clone 133/134 at 0.01mM IPTG and 200ug/mL arabinose). The expression vectors pecan135 codes for g6p, pecan136 codes for g7p, and pecan137 codes for g8p.

### Example 2F

Monoclonal phage ELISA was performed on Marburgvirus NP to compare conventional display of MBG B sdAb (pecan126 XL1-Blue), HBV88 mediated display (pecan126 HBV88), separate transdisplay components (pecan133 HB2151 and pecan134 HB2151) and combined transdisplay components (pecan133+134 HB2151). Signals on negative control Ebolavirus NP antigen ranged from 0.017 to -0.007.

**FIG. 2F** reveals that when in trans-displayed mode with 10 µM IPTG and arabinose, the sdAb is displayed. Positive control pecan126 vector in either XL1-Blue or HBV88 was used to benchmark transdisplay. Pecan133, 134 and 133+134 were employed in HB2151, such that 133 expresses just periplasmic sdAb, 134 expresses supEV88-core-streptavidin-g3p and 133+134 expresses both and results in sdAb display. (Signals are specific for the Marburg nucleoprotein since simultaneous probing on Ebola virus NP yielded ranges of 0.017- 0.007).

Advantages of transdisplay are that with a single library of sdAb (or other ligand) in pecan133, resulting clones can be mobilized rapidly to any streptavidin based display system without the need for subcloning. Such systems may enable multicopy to single copy display on phage by employing g8p rather than g3p as the streptavidin carrier to mimic high avidity low affinity to low avidity/high affinity selections. Importantly, it is not limited to phage display and may well also allow cell surface display via fusions of streptavidin to lpp-ompA or ICE nucleation protein, etc. An advantage of the transdisplayed system will be the ability to display complex proteins that are not easily amenable to direct display, and that the final level of displayed protein will more accurately reflect the amounts occurring in the periplasm.

### Example 2G and 2H

Small-scale shockates from constructs and strains in soluble sdAb expression were paired to demonstrate that the transdisplay vector pecan133 is equivalent to the conventional display vector pecan126 whether in HB2151 or HB2151+pecan134.

**FIG. 2G** reveals that when in soluble sdAb mode following 1 mM IPTG induction, the rapid ligand pairing system generated from pecan133 is functional on par with previously engineered pecan126 whether on its own in HB2151 or with pecan134. Vector pecan133 expresses sdAb-BAP and Maeda birA, vector pecan134 expresses the supE-streptavidin-g3p fusion only (i.e. no sdAb), pecan126 and pecan114 are positive and negative controls since both express the sdAb but 126 has the BAP tag and 114 does not. **FIG. 2G** shows the results when assayed with Marburg virus NP surrogate antigen and **FIG. 2H** with negative control Ebola virus NP antigen.

### Examples 2I and 2J

**FIGS. 2I** **and** **2J** shows the examination of single monoclonal clones from the polyclonal response shown in **FIG. 1T** for pecan133/134. Here, although the signals are relatively weak compared to 126 (since it is not as effective at display), there are clearly some specific clones here, like the clone in well A5. As expected, when matched in the pairing system the OD are on a par with pecan126. **FIG. 2I** is the dataset for the monoclonal phage ELISA on plate coated BoNT A and **FIG. 2J** is the dataset for the same ELISA performed on ovalbumin.

### Example 2K

Pairing of sdAb clones identifed from pecan133/134 retrofitted heptaplex BoNT immune library following selection and screening reveals several promising and novel combinations of sdAb capable of recognizing 1 µgmL-1 BoNT serotype A at equivalent overall intensities as the HBV88 setup (compare **FIG. 2K** with **FIG. 1J**). Negative control antigen performed alongside showed no background signals (**FIG. 2L**). This dataset demonstrates that pecan133/134 based display can serve as a productive resource for repertoires, enabling them to be rapidly deconvoluted and resulting clones paired.

### Example 2L

Negative control antigen ovalbumin 1 µg/mL used alongside the experimental pairing reveals no background signals after pairing sdAb clones isolated from the pecan133/134 mediated retrofit of a heptaplex BoNT immune library (**FIG. 2L**).

### EXAMPLE 3

### Applying transdisplay and pairing to a single pot library.

We next sought to apply transdisplay and pairing to a single pot library to determine if we could truly accelerate immunoassay formulation through bypassing immunization. Since the semi-synthetic llama sdAb library "Nomad#1" had proven successful in generating a specific and sensitive sdAb to both Marburgvirus and Ebolavirus nucleoproteins using conventional sdAb-g3p panning, we retrofitted it for transdisplay to compare and contrast clones selected using the new approach. The library was made in a hygromycin resistant version of pecan133 (pecan164) to eliminate possible enrichment of existing sdAb in ampicillin display vectors and then selected on Ebolavirus Zaire (strain Kikwit 1995) at BSL-4. Polyclonal phage ELISA showed enrichment of an Ebolavirus specific population (FIG. **3A**). 22 of 22 clones picked from rounds 3 and 4 were sequences of a clone (EBOZ C) that we had isolated previously at a frequency of 11/26 (predicted amino acid sequences shown in **FIG. 3B**). Importantly, EBOZ C had never been individually mobilized to any pairing, transdisplay or hygromycin based vector and could only have arisen through enrichment from the retrofitted single-pot library. The failure to isolate five other previously identified EBOZ sdAb clones could be due to their absence in the retrofit since it only matched the original library size and/or the log decrease in effectiveness of transdisplay observed in **FIG. 2F****.** The EBOZ C clone was directly employed via the dual use host HB2151+pecan134 in a sdAb captor/phage tracer Filovirus titration (**FIG. 3C**) and a matching sdAb captor/sdAb tracer pairing (**FIG. 3D**). While phage tracer yielded a relatively sensitive assay capable of detecting hundreds of pfu, the sdAb pairing struggled at these low antigen concentrations reflecting the decrease in signals visualized on surrogate antigens previously for MBGB sdAb in **FIG. 1C** and **FIG. 1D****.** This is a demonstration of a transition from a single pot library to an antigen capture assay without antibody purification. Further optimization of the transdisplay system or application of the conventional display vector still with pairing capability (pecan126) may enable the recovery of all of the previously isolated clones. Additionally, access to higher quality libraries and diversity, while retaining solubility or assembly of a larger sdAb repertoire based upon more donors, may well yield a broader diversity of more sensitive clones for immediate use on low virus numbers.

### Example 3A

**FIG. 3A** shows enrichment of polyclonal phage specific for Ebolavirus Zaire (EBO) rather than Marburgvirus Musoke (MBG) through four rounds of panning the semi-synthetic llama sdAb library Nomad#1 within HygR pecan133 (pecan164) on Ebolavirus Zaire virus.

### Example 3B

**FIG. 3B** show the predicted amino acid sequence of the anti-Ebolavirus Zaire clone isolated from transdisplay of the retrofitted single-pot library Nomad#1, compared with the original sequence isolated via conventional panning.

### Example 3C

The single sdAb clone within HB2151+pecan134 (EBOZ C) was produced as shockate for captor and phage as tracer to generate an Ebolavirus Zaire (EBO) specific assay, while MBGB captor and phage from HB2151+pecan134/133 served to confirm Marburgvirus (MBG) control was present

### Example 3D

Performed side by side with the phage tracer experiment in Example 3C on the very same dilutions of virus, we find using sdAb as both captor and tracer reveals that at these low virus numbers the system can struggle for sensitivity without phage amplification.

### Example 4

Another embodiment of the method would be screening for improved solubility variants. For example, an antibody clone maybe first screened to ensure binding has been retained. By replacing the g3p display portion of the expression vector pecan126 with mature β-lactamase, one can select for mutant affinity agents with improved solubility by increasing ampicillin concentrations in HBV88 in display mode. By switching to soluble mode, one then has the direct means to screen any surviving clones for binding using a template similar to **FIGS. 1Q** and **1R** and **FIGS. 2G** **and** **2H****,** aiming to generate high signals using increasingly smaller amounts of shockate as captor and tracer and it is very convenient to be able to switch from fused to unfused protein for enzymatic assay and characterization. Vectors assembled for this task are shown in **FIG. 4A** where a basal level of β-lactamase expression afforded by the sdAb fusion (pecan148) gives one an estimate of the gain SBP and HRP must achieve in order to become useful genetic fusions in pecan148 HRP and pecan148 SBP. Pecan146 is a control vector with no β-lactamase resistance and pecan150 is an example of the maximal level of β-lactamase resistance when no upstream motif is present, especially useful for the evolution of better sdAb solubility rather than HRP and SBP that would most likely be too much gain in one round of *in vitro* evolution. These enzymes require heme to fold productively and the outer membrane of *E. coli* enables diffusion of this to the periplasmic compartment to enhance productive expression.

The reagents and methods described before for selecting polypeptides with greater binding affinity, can be used to select for improved solubility. For example in **FIGS. 4B****-4D,** phagemids were propagated in HBV88 overnight at 30°C and a loop streaked out onto 2xTY 2% glc plus Cm30 Hyg200. **FIG. 4B** is an illustration describing the vector construct contained in the host cells in each of the five sections of the plates. 10 µM IPTG is present in all plates. **FIG. 4C** shows cells growing in the presence of arabinose 2000 at different concentrations of ampicillin and **FIG. 4D** shows cells growing in the absence of arabinose at different concentrations of ampicillin. In both **FIG. 4C** and **FIG. 4D****,** the plates have different concentrations of ampicillin to measure how much soluble protein is produced. In both **FIG. 4C** and **FIG. 4D****,** the plate in the top right quartet has no ampicillin, the plate in the top left quartet has 25 µg/mL of ampicillin, the plate in the bottom right quartet has 100 µg/mL, and the plate in the bottom left quartet has 400 µg/mL.

In the absence of ampicillin all constructs grow, even control pecan146 which has no Bla gene onboard. At ampicillin concentrations of 25 µg/mL and higher, the pecan146 does not grow whether arabinose is present or not. The other constructs show enhanced growth when arabinose is supplied, favoring read-through of the downstream Bla gene. Pecan150 still forms a complete streak at 400 µg/mL of Amp in ara2000 but struggles at 100 µg/mL in the absence of arabinose. Pecan148 forms a complete streak at 100 µg/mL of Amp in ara2000 yet struggles at amp 25 µg/ml in its absence. The HRP and SBP versions of 148 struggle to encode resistance to 25 µg/ml amp in the absence of arabinose yet manage this in ara2000 and partially at 100 ug/mL. These plates are not streaked from minimal medium cultures, nor washed and so will likely contain arabinose analogues that can give rise to the leaky expression in the absence of additional arabinose in the plate.

By plating a library of horse radish peroxidase (HRP) or soybean peroxidase (SBP), mutants on ara2000 and amp400 may be selected for variants that encode increased Bla resistance. By then growing survivors in liquid medium in the absence of arabinose, soluble unfused proteins can be analysed for enzymatic activity (HRP and SBP for peroxidase activity). Here the sdAb serves as a calibration molecule. Fusion products like sdAb-HRP and sdAb-SBP may function as heat stable diagnostic reagents. In another embodiment, the single domain antibody may be evolved using a pecan126/148 hybrid where g3p (on a HygR backbone) is replaced by Bla yet the BAP is retained to enable rapid screening of the ability to bind antigen as a sandwich.

### Example 5

The following methods, materials, and procedures are intended to be exemplary and are not intended to limit the scope of the invention.

General. Recombinant DNA methods were performed according to established procedures and employed commercially available reagents, including but not limited to restriction enzymes and β-agarase (New England BioLabs, Beverly, MA); T4 DNA ligase, CIP and T4 PNK (Roche, Nutley, NJ), AgarACE (Promega, Madison, WI); GTG low melting temperature agarose, (Lonza, Walkersville, MD); oligonucleotides (Integrated DNA Technologies, Coralville, IA); synthetic core streptavidin gene (Genscript,Piscataway,NJ). HotStart YieldAce, (Stratagene, La Jolla, CA) was used for PCR amplification unless otherwise noted. Assemblies involving PCR amplification or oligonucleotide bridging were sequenced through the inserts and junctions to verify the desired construct. Cloning was typically in XL1-Blue unless otherwise stated, with constructs under study mobilized to hosts under investigation. Expression/display cultures were not supplemented with additional biotin.

Biosafety. Select agent work was carried out at Texas Biomedical Research Institute following all federal guidelines as part of the CDC Select Agent Program and with local Biohazard and Safety Committee approval. Botulinum neurotoxins were handled in enhanced BSL-2 conditions while Filoviruses were handled in the full suit BSL-4 laboratory.

Expression and display vectors. Non-biotinylating control vectors: pecan45 was modified to pecan73 by exchange of the *lac* for *tac* promoter using technique of SOE-PCR (splicing by overlap extension by the polymerase chain reaction), and insertion of PCR amplified MBG B sdAb, specific for Marburgvirus nucleoprotein, from pecan21LgMBGB to delete the hinge and fuse framework four TVSS directly to the *Not*I site AAA reading frame to provide a basal hingeless pel-sdAb-His6 expression cassette. The full length g3p gene was PCR amplified from amberless pecan21 using primer NotHisamberTEV(5'-gaattc,gcg,gcc,gca,cat,cac,cat,cac,cat,cac,tag,ggt,ggc,ggt,gga,tct,gag,aat, ctt,tat,ttt,cag,ggc, gga,ggt,ggc,ggt,gct,gaa,act,gtt,gaa,agt,tgt-3') encoding His6 tag, amber codon plus a TEV protease cleavage site, and back primer g3pTAAHind3 (3'-tat,gac, gca,tta,ttc,ctc,aga,att,att,cgaaaaaa-5'), and inserted into the *Not*I and *Hind*III sites of pecan73 to provide the basal phage display vector pecan114. The g3p gene was sequenced using primers lacZ' (5'-ctatgaccatgattacgaatttctag-3'), AHX228 (5'-cttatatcaaccctctcgacggc-3'), AHX229 (5'-tcgtttgtgaatatcaaggcc-3') and AHX230 (5'-cattggtgacgtttccggcc-3').

Biotin acceptor peptide insertions: Pecan122 was made by replacing g3p from pecan 114 with a re-amplified product using front primer *Not*BAPHisg3p (5'-ca,gcg,gcc,gca,gga, ggc,ggt,gga,tct,ggc,ctg,aac,gat,att,ttc,gaa,gct,cag,aaa,atc,gaa,tgg,cac,gag,ggc,ggt,gga,ggc,tct,c at,cac,cat,cac,cat,cac,tag-3') and back primer g3pTAA*Hind*3 to incorporate the minimal BAP32 (underlined) flanked by Gly4Ser coding sequences. BAP linker variants employed a similar strategy but switching out the front primers with ones lacking the front (NotBAPG4SHisg3p), back (NotG4SBAPHisg3p) or both (NotBAPHisg3p Nolink) Gly4Ser coding sequences. The g3p and linker sequences were verified as before.

Biotin ligase gene insertions. The birA second cistron was PCR amplified from 1 µL of an overnight culture of *E. coli* DH10B, inserted into the *Hind*III site of pecan122 and orientation mapped wrt internal *Pst*I and the framework 1 *Pst*I of the sdAb. The front primer for full-length birA was H3birAFOR (5'-aaaagcttaggaggacagctatg,aag,gat,aac,acc,gtg,cca-3') and for the deletion mutant34 was H3MaedaFOR (5'-aaagcttaggaggacagctatg,atc,cag,tta,ctt,aat,gct,aaa,c-3') initiating to Ile64 with the T7 gene 10 ribosome binding sites underlined. For the low expression versions, the ATG initiation codon was changed to GTG to encode H3birAgtgFOR and H3MaedagtgFOR respectively. The common back primer birAH3BACK (3'-g,gac,gca,tca, cgt,ctt,ttt,att.ttcgaaaa-5') was used for all birA formats. The various birA cistrons were sequenced with AHX89 (5'-cgcagtagcggtaaacggc-3') and AHX230.

Direct supE expression vectors. The starting supE tRNA sequence was initially synthesized as two complementary oligonucleotides supE top (5'-gtggggtat cgccaagcggtaaggcaccggattctaattccggcattccgaggttcgaatcctcgtaccccagccaa-3') and supE bottom (5'-agctttggctggggtacgaggattcgaacctcggaatgccggaattagaatccggtgccttaccgcttggcgataccccactgca-3') based upon Genbank M1070835. These were phosphorylated, annealed and bridged into *Pst*I / *Hind*III digested pAR3, a p15a origin based chloramphenicol resistant arabinose inducible construct (a generous gift of Dr. Julio Gutiérrez) to make pAR3supE. Low copy number of the vector dictated PCR amplification of the inserts by flanking primers and subsequent sequencing of the PCR product. pSCUPER was assembled by ligating native Pfu polymerase (Stratagene) amplified segments of both pSC101 and pAR3supE in PEG ligation buffer in the presence of T4 polynucleotide kinase. Segments were generated as follows: pSC101 (ATCC, Manasas, VA) was first amplified using PacPSC101BACK (5'-aaattaattaagacagtaagacgggtaagcc-3') with E93Rbottom (3'-ccaattccgaaagcctaaaggtcacctg-5'), and PacPSC101FOR (5'-cccaattaattatgattttttccccacgggag-3') with E93Rtop (5'-ggttaaaggctttcggattttccagtggac-3'). The products were then fused by SOE-PCR using the Pac primers for pull-through to create a 2.1 kbp high copy number pSC101 origin12 segment. The pAR3-supE was amplified using PacPAR3BACK (5'-aaattaattaacctgaagtcagccccatacg-3') and PacPAR3FOR (5'-cccaattaattccgaataaatacctgtgacgg-3') to create the 2.0 kbp arabinose inducible supE segment bearing the chloramphenicol resistance gene. Here, sequencing was replaced by phenotypic screening in two ways. First, clones were benchmarked against the original high copy number pSC101 mutants (generous gifts of Dr. Gregory Phillips) for approximate copy number equivalence by agarose gel electrophoresis. Second, clones were mobilized to DH10F'tet with pecan114 to screen for suppression in the presence of 2% glucose, 2000 µgmL-1 arabinose and 1 mM IPTG following superinfection with M13K07 and kanamycin selection. Suppression to enable MBG B sdAb display was monitored by phage ELISA with positives scored for signal on Marburgvirus nucleoprotein and not Ebolavirus nucleoprotein, and the highest signal to noise clone (#9) taken forward. pSCUPERV88 and pSCUPERV89 were made by replacing the wild type supE region between the *Pst*I and *Hin*dIII sites of pSCUPER with phosphorylated and annealed bridges comprising either V88 top (5'-gtggggtatcgccaagcggtaaggcaccggattcta actccggcattccgaggttcgaatcctcgtaccccagccaa-3') plus V88 bottom (5'-agctttggctggggtacgag gattcgaacctcggaatgccggagttagaatccggtgccttaccgcttggcgataccccactgca-3') or V89 top (5'-gtggggtatcgccaagcggtaaggcaccggattctaaatccggcattccgaggttcgaatcctcgtaccccagccaa-3') plus V89 bottom. (5'-agctttggctggggtacgaggattcgaacctcggaatgccggatttagaatccggtgccttaccgcttggcgatacccc actgca-3').

Transdisplay platform vectors. The g7p, g8p and g9p genes were amplified from M13K07 helper phage replicative form in a similar manner to the g3p gene using front primers NotHisAmberTEV-g7p, -g8p and -g9p which had the common 5'-region encoding NotI-His6-amber-TEV but varied in their priming sequences according to the gene targeted; g7p-atg,gag,cag,gtc,gcg,gat,ttc-3'; g8p - gct,gag,ggt,gac,gat,ccc-3'; g9p - atg,agt,gtt,tta,gtg,tat,tct,ttc. The back primers were g7pTAA*Hind*3 (3'-t,tag,cga,ccc,cca,gtt,cct,att,att,cgaaaaa-5'), g8pTAA*Hind*3 (3'-tgg,agc,ttt,cgt,tcg.att.att.cgaaaaaa) and g9pTAA*Hin*d3 (3'-gg,gca,aat,tac, ctt,tga,agg,agt.att.att.cgaaaaaa-5'). The amplification products were used to replace g3p in pecan114 *via Not*I and *Hin*dIII and sequenced with primer lacZ' to create pecan115 (MBGB sdAb-gp9), 119 (MBGB sdAb-gp7) and pecan120 (MBGBsdAb-g8p). A synthetic *E. coli* optimized core streptavidin gene from Glu13 to Ser139 with flanking *Nco*I (encoding AMA) and *Not*I (encoding AAA) sites and based upon Genbank X03591.137 was used to replace the sdAb in pecan114, 115, 119 and 120 to make the strep-display versions with clones sequenced using lacZ' primer.

Strep-display cassettes were PCR amplified using PstV88lacZ (5'-taactgcagtggggtat cgccaagcggtaaggcaccggattctaactccggcattccgaggttcgaatcctcgtaccccagccaatttattcaagacgcttaccttg taagtgcacccagtctatgaccatgattacgatct-3') which encodes the V88 supE mutant and intervening region between the two Gln2 tRNAs38 and the common back sequencing primer AHX89 from respective strep-gp vectors and used to replace the resident supEv88 insert within pSCUPERV88 via *Pst*I and *Hind*III. The starting constructs are the strep display vectors that fuse the strep minigene to one of the minor coat proteins, and the result are matching strep display vectors where the transcript is headed by a cleavable V88 supE tRNA sequence.

Transdisplay sdAb vectors. The pecan133 vector was derived from pecan126 by replacing the resident g3p and GTGΔbirA cistron region *via Not*I and *Hind*III with a re-amplified GTGΔbirA region made with Not126Asc (5'-tca,gcg,gcc,gca,gga,ggc,ggt, gga,tct,ggc,ctg,aac,gat,att,ttc,gaa,gct,cag,aaa,atc,gaa,tgg,cac,gag,ggc,ggt,gga,ggc,tct,cat,ca,cat ,cac,cat,cac,taa.taa.ggcgcgccttaggaggacagctgtg,atcc-3') and AHX89 which fuses the G4SBAPG4S-His6 region to the *Not*I site and two ochre termination codons and re-encodes the ribosome binding site and GTG initiation region for ΔbirA.

Hygromycin resistant sdAb transdisplay vector. The hygromycin gene was a generous gift of Dr. Jim Sweigard and was isolated from pCB1004 by ligation in PCR using HygFRONTSwa (5'-aaggaaatttaa.atg,aaa,aag,cct,gaa,ctc,ac-3'), HygBACKSwa (3'-ca,ggc,tcc, cgt,ttc,ctt,atttaaatttatccgt-5'), HygNcoDEL (5'-cag,ccg,gtc,gcg,gag,gcT,atg,gat,gcg,atc,gct,g-3') and HygNdeDEL (5'-ca,tgg,cgt,gat,ttc,atC,tgc,gcg,att,gct,ga,cc) to delete internal *Nco*I and *Nde*I restrictions sites and inserted in place of the ampicillin resistance gene in pecan133 *via Swa*I to make pecan164.

Expression of sdAb. Small-scale cultures of 20 mL terrific broth (no glucose plus appropriate antibiotics: ampicillin 200 µgmL-1 for most pecan vectors, 200 µgmL-1 hygromycin B for pecan164, 30 µgmL-1 chloramphenicol for pecan134) were seeded with 400 µL of a saturated overnight 3.5 mL culture, grown with vigorous aeration for 2h at 30°C, 0.5h at 25°C, induced with addition of IPTG to 1 mM and shaken for a further 3h. Cells equivalent to approx. 20 OD at A600 nm cm⁻¹ were harvested as described previously using a scaled down version of the Neu and Heppel method, to yield approx. 1 mL volume of osmotic shockates, which were either used immediately or stored at -20°C until required. Mid-scale expression with subsequent IMAC and gel filtration was performed on 400 mL scale cultures.

Mass spectrometry. Samples were analyzed by Kevin Hakala and Dr. Susan Weintraub at the Institutional Mass Spectrometry Laboratory, UT Health Science Center San Antonio, using ESI Infusion on the ThermoFinnigan Quantum Triple Quadrupole Mass Spectrometer. About 5 to 10 µg of protein was diluted ∼10 fold into 0.2% TFA (10 µl ptn into 100 µl TFA). The sample was bound to a C4 ZipTip (Millipore) by aspirating and dispensing into a second tube labeled "flow through" ∼6 times, 20 µl each. The C4 ZipTip was then washed with 100 µl 0.2% TFA, and eluted into 50 µl 50% acetonitrile, 0.5% acetic acid solution. The eluate was infused into a ThermoFinnigan Quantum triple quadrupole mass spectrometer at 0.6 µl/min via a 250 µl SGE glass syringe using the mass spectrometer's integrated syringe pump. A New Objective IntegraFrit (IF360-75-50-N-5) was used inline as a pre-filter before the sample reached the New Objective Column Adapter (ADPC-IMS), which replaces the standard ESI probe of the ThermoFinnigan Ion Max source, and introduced into the mass spectrometer via a New Objective PicoFrit (PF360-75-10-N-5) emitter. The instrument was set in positive ion mode. Spray voltage was 2000V, and the heated capillary was set at 250°C. Data was obtained using the full scan mode of quadrupole 1. Repeated snapshot scans were acquired from 150-1500 m/z. Q1 peak width was set between 1.2 and 4.0 depending upon the sample molecular weight and data processing was set to average between 20 to 50 spectra depending upon the peak intensity. Data files were opened in ThermoFinnigan's QualBrowser (v 2.0) and processed by using 'boxcar' type smoothing set at 7 points. The exact mass spectra was then exported to the clipboard and subsequently opened in ProMass for Xcalibur (v 2.5 SR-1) for deconvolution. Baseline removal was set at low/normal, and smoothing was turned off. Comprehensive deconvolution settings were as follows: peak width 3; merge width 0.3; minimum score 0.2; normalize score 1. Result files were subsequently captured as screenshots for record.

Antigens. Arrays of Marburgvirus NP offering a potentially polyvalent surrogate have been demonstrated previously when overexpressed in the *E. coli* cytosol. An untagged Musoke NP gene and Ebola Zaire strain Kikwit 1995 gene were assembled, and overexpressed using pecan42 in Tuner+pRARE. Immunoblotting and probing with sdAb-AP fusions were first used to verify small-scale expression which was then scaled up to 2x400mL in glucose free terrific broth with induction for 6 h at 1 mM IPTG at 25°C, yielding approx. 20g wet weight total. Pellets were resuspended in total volume of 100 mL for cell lysis via bead-beating in 100 mM TrisHCl pH7.5, 1 mM EDTA, Roche complete protease inhibitors, 0.05% Tween-20 and 1 mM DTT. Lysates were clarified by repeated centrifugation and decanting (Allegra GPR, 5.75 krpm, 30 min, 4°C), and finally filtration through a 0.22 µm vacuum filter and stored in 2 mL aliquots at -80°C until required. ELISA titration of passively immobilized sdAb as captor and sdAb-AP as tracer in Tris buffered saline was used to verify that the antigens were a suitable crude surrogate for polyvalent viral NP with 10 µL per well in MPBS determined to be our standard.

Nucleoproteins were produced for direct capture ELISA using a similar harvesting approach as applied to the C-terminally His tagged proteins in pecan42 to enable immobilized metal ion affinity chromatography (IMAC) purification of denatured monomer. Beadbeating to lyze the cells was carried out 100 mM TrisHCl pH 7.5, 100 mM NaCl, 5% glycerol, 6M guanidine HCl, 0.05% Tween-20, 10 mM β-mercapoethanol and 20 mM imidazole with protease inhibitor cocktail. Clarified and filtered supernatant was applied with a superloop to a 1 mL nickel sepharose FF column and protein eluted via a 20 mM to 600 mM imidazole gradient. Peak fractions were silver stained and western blotted with anti-His HRP revealing expected 100 kDa band as the dominant species. These fractions were pooled and stored in 100 µL fractions at -20°C. Prior to use after thawing, the denatured preparation was microfuged to remove most of the aggregates that formed, with ELISA plate coating employing 100 µL of a solution of 1µL of the clarified nucleoprotein supernatant per mL PBS overnight at 4°C. Botulinum neurotoxins were purchased from Metabiologics (Madison, Wisconsin). 1 µgmL-1 in PBS was employed for panning, ELISA coats and pairing.

Ebolavirus Zaire strain Kikwit 19956 was amplified in 16 x 225 cm³ flasks of Vero cells in DMEM/5% FBS/penicillin/streptomycin for 4 days. The 40 mL supernatants were collected, gently clarified by centrifugation (Allegra 6R, swing-out, 2.5 krpm, 5 min, 4°C) and stored at -80°C. Fresh media was added to the flasks and the amplification mixture was left for four more days. Day four supernatants were thawed at room temperature, day eight supernatants were gently clarified by centrifugation and the virus was precipitated by addition of 1/5th volume of PEG 8000/2.5M NaCl at 4°C overnight. Precipitated virus was pelleted (Allegra 6R, 3.5 krpm, 15 min, 4°C), resuspended in a total of 4 mL PBS, and 4 x 1 mL centrifuged through 10 % (w/v) sucrose/PBS onto 65 % sucrose cushions (Beckman L70M , SW40, 20 krpm, 1 h, 4°C). Virus was harvested, diluted to 6 mL and loaded a top six 20 to 65% continuous gradients, and centrifuged at 20 krpm for 24 h at 4°C. Bands were made visible with a torch from above, harvested, pooled and dialyzed against 3 x 300 volume changes of PBS before aliquoting and storage at -80°C. Virus was titrated by plaque formation on duplicate 6 well plates of Vero cells with a one hour infection, gentle wash and overlay with 2mL of EMEM/5% FBS/ penicillin/ streptomycin containing 0.6% Seaplaque GTG agarose. On day ten, 2 mL of 4% formaldehyde was added to each well. The plates were incubated in a closed box at 37°C overnight. The agarose plugs were removed and the monolayer was stained with crystal violet for plaque visualization and counting. Coats of virus for the first round of panning were 8 x 100 µL each containing approximately 4e+5 pfu. Coats for rounds two through four were 8 x 25 µL virus + 75 µL PBS each containing approximately 1e+5 pfu. For polyclonal phage screening, wells were coated with approximately 4e+3 pfu and for monoclonal phage screening wells were coated with approximately 1e+4 pfu. Marburgvirus strain Musoke 1980 purified previously was used as the control virus. Viruses were incubated in a final concentration of 0.1% Triton in MPBS for 10 min before applying to the ELISA plate to release the nucleocapsid.

ELISA pairing of sdAb. 100 µL of neutravidin (Pierce, Rockford, IL) at 1 µgmL⁻¹ in PBS was used to coat a 96 well high binding ELISA plate, either clear #3590 or white #3922 (Corning Costar, Tewksbury, MA) overnight at 4°C. The plate was washed 3 times with PBS, and blocked by addition of 375 µL of PBS + 2% BSA + 0.1% Tween-20 (PBSBT) for minimum of 1 h at room temperature. The block was replaced by 100 µL of fresh block containing the desired volume of shockate, typically 1 µL or 10 µL and left either overnight at 4°C or shaking (Barnstead Lab-Line plate shaker setting #2) at room temperature for 30 min to enable the neutravidin to capture the sdAb. The plate was washed 3x with 175 µL of PBS + 0.1% Tween-20 and 2x with PBS. 100 µL volumes of antigen were added in PBS + 2% non-fat dried Carnation milk (MPBS) and the plate shaken for 15-30 min. The plate was washed as before, the tracer (typically 1 µL of shockate) in 100 µL volume of PBSBT was added and the plates shaken for 15-30 min. The plate was washed as before and 100 µL of high sensitivity neutravidin HRP conjugate (Pierce) at 1 in 10,000 dilution in PBSBT was added, and the plate shaken for a further 15-30 min. The plate was washed as before and 100 µL of TMB-ultra (Pierce) added and stopped with 50 µL of 0.5 M H₂SO₄ typically after approx. three minutes, or the plate was developed with Femto ELISA chemiluminescent substrate (Pierce) and read on a luminometer (Turner Biosystems) with a 2s integration.

Phage display and ELISA. For conventional display and vector proving, 400 µL of overnight cultures of phagemids in XL-1Blue/DH10-tetF' were used to inoculate 40 mL 2xTY 2% glucose plus appropriate antibiotics in 250 mL baffled flasks and shaken for approx. 2h at 37°C until an O.D. of 0.4-0.6 was reached. M13K07 at a multiplicity of infection (moi) of 20 was mixed in, the culture left static for 30 min, and then shaken overnight at 30°C in the presence of 70 µgmL⁻¹ kanamycin and 10 µM IPTG. Display using HBV88 employed 10 µM IPTG and 2000 µgmL⁻¹ L-arabinose while display in HB2151+pecan134 utilized 10 µM IPTG and 200 µgmL⁻¹ L-arabinose. 2 mL aliquots were clarified by centrifugation (13,500 rpm, 10 min) and stored at -20°C until required. Detection of phage in sdAb captor or direct phage ELISA on antigens was using standard methods and anti-M13-HRP conjugate. Phage ELISAs were generally performed in MPBS except the capture of strep-phage on biotinylated sdAb which was in PBSBT to avoid competition from biotin in milk.

Library Retrofitting. The previously assembled single-pot and immune library were stored as both phage and glycerol stocks, the latter containing enough representations to seed 6 x 400 mL cultures to an OD of 0.1 for re-rescue, i.e. approx. 1.2 e+11 cfu. One 2 mL glycerol was diluted to 24 mL with media and divided into 12 x 2 mL and miniprepped (Qiagen) for elution of approx. 12 x 10 µg in 80 µL of EB. The destination vectors pecan126, pecan133 and pecan164 were modified by replacement of the MBG B sdAb with a 2 kbp tetracycline resistance gene fragment from pecan21 via *Nco*I (partial)/ *Not*I to provide a convenient marker for scoring positive sdAb gene inserts and to enable *Sfi*I/*Sfi*I cloning7 for the single-pot retrofit while immune libraries employed *Sfi*I/*Not*I*.* Each vector was grown in 12 x 3.5mL of terrific broth, 2% glucose and appropriate antibiotics and 12 x 2mL miniprepped for elution of approx. 12 x 10 µg in 80 µL EB which were then pooled. Vectors and inserts were digested by addition of 120 µl 10 x React2 buffer, 120 µL 10 x BSA and 24 µL of *Sfi*I (20 U/µL) and left for 12-18 h in a 50°C oven. The immune library inserts and vectors were further digested by addition of 60 µL React 3 and 24 µL *Not*I (10 U/µL) for a further 12 h at 37°C. Vector digests were dephosphorylated by addition of 120 µL 10xCIP buffer and 40 µL CIP (1 U/µL) and left at 37°C for 2h (although self-ligation is not an issue here, we dephosphorylate to reduce inter vector-vector ligation reducing the available destination DNA for the insert). DNA was electrophoresed on 1% (for insert) or 0.5% (for vector) GTG TAE gels and the appropriate bands excised in a volume of about 2.5-3.5 mL for a scaled-up version of in agarose ligation. The gels were melted at 70°C then kept at 37°C and both vector and insert were added and mixed in 400 µL aliquots into 6-8 tubes each containing 780 µL water, 200 µL 10xT4 DNA ligase buffer (Promega) and 20 µL β-Agarase (1U/µL) which stayed liquid at room temperature (approx. 75F). 20 µL of T4 DNA ligase (1U/µL) was added, the tubes mixed gently by inversion, covered in foil and left at slightly warmer than room temperature atop the hybridization oven for 18-24h. The ligations were aliquoted to 24-32 x 500 µL, each extracted with 450 µL of phenol/chloroform (Invitrogen), and the 450 µL supernatants precipitated by addition of sodium acetate/ethanol and left on the bench for 2h only to avoid excessive salt precipitation. Tubes were microfuged at 13.5 krpm for 15 min, supernatants poured off, pellets briefly washed with 350 µL 70% EtOH, recentrifuged for 5 min, aspirated and dried briefly in a tissue culture hood. Each pellet was resuspended in 42-32 µL (depending on the number of ligations) and pooled to provide a combined 32 aliquots of 60 µL for bulk electroporation which were aliquoted for storage at - 80°C until required.

HBV88 or HB2151+pecan134 were made electrocompetent using low temperature growth in low salt YENB media growth followed by extensive washing and reliably yielded transformation efficiencies in the mid 1e+9 cfu/µg ccc pUC19 range only some 2-4 fold less than home-made preparations of high efficiency strains such as DH10B or DH10F'tet. A streak on M9/chloramphenicol + thiamine minimal agar to select the F'-episome was rinsed into 400 mL of liquid M9 equivalent and shaken overnight at 37°C. From this starter, 6x400 mL flasks of YENB plus chloramphenicol were inoculated to an OD600nm of 0.05 cm-1 and shaken at 25°C until an OD of between 0.4 and 0.5 was reached (approx. 6-7 h). Cells were immediately pelleted (Allegra GPR, 4x750 mL pots, swing out rotor, 4°C, 20 min), resuspended gently in a total volume of approx. 1.5 L ice cold water and re-pelleted (Allegra, 6x250 mL, fixed angle, 5.75 krpm, 20 min 4°C). Cells were washed in water once more, then washed in 15% glycerol and finally resuspended in 8.7 mL of 15% glycerol and aliquoted to 32 x 270 µL, snap frozen in -80°C isopropanol and stored frozen until required.

Electroporations of the 60 µL ligation and 270 µL cell aliquots were performed using an electroporator (BioRad) at 2.5 kV with 2mm gap electrocuvettes (Bulldog Bio Inc., Portsmouth, NH) that accommodated the combined volume. Following electroporation the cuvette contents were poured into 50 mL Falcon tubes followed by 3 cuvette washes of 2 mL of prewarmed (37°C) SOB 2% glucose with two electroporations worth pooled in each Falcon tube. The mixes were left static for 60 min and cells were then pelleted (Allegra GPR, 3 krpm, 10 min, 20°C), resuspended in 400 µL supernatant and spread on Bioassay dishes containing 250 mL 2xTY 2% glucose with appropriate antibiotics. Following growth overnight, cells were scraped with a 3 inch wide wallpaper scraper (Hyde Tools) to a pot and the plates also washed with 4 mL of 2xTY 2% glucose which was added to the pot and the whole lot mixed thoroughly. After measuring the OD600nm*, 6 flasks of 400 mL 2xTY 2% glucose were seeded to and OD600nm of 0.05, grown with shaking at 37°C to an OD of 0.4-0.5 and infected with M13K07 at an moi of 20 for 1 h static. Kanamycin, IPTG and arabinose were added according to the system employed and rescue proceeded for 18-24h at 30°C. Cultures were clarified by centrifugation (Sorvall RC6+, 4x1 L, 8 krpm, 1 h, 4°C), pooled and precipitated by addition of 480 mL of 20% PEG6000/2.5 M NaCl and overnight stirring at 4°C. Phagemids were pelleted (Allegra GPR, 3.75 krpm, 4x750 mL swing out, 1h, 4°C), drained and resuspended in a total volume of 16 mL PBS to which was added 16 mL of glycerol, and 16 x 2 mL aliquots stored at -80°C until required. *The remainder of the cell suspension was combined with an equal volume of ice cold 30 % glycerol in terrific broth and aliquoted into 40-50 2 mL cryovials such that each aliquot could seed another six flasks if required.

Library Selections. Conventional panning methods were used for these targets as described in the prevailing literature. An 8 well strip was coated with 8x100 µL of antigen overnight at 4°C. Wells were washed 3x with 175 µL PBS and blocked for 1 h with 350 µL MPBS. Phage representing 100 clones of each library were applied in 8x100 µL MBPS for 30 min shaking, wells washed with PBST and PBS before 8x100 µL triethylamine elution for 10 min followed by pooling and neutralization with 400 µL 1M Tris-HCl pH 7.5. For BoNT A, the single round of panning was 20 washes each and for Ebolavirus Zaire it was 10, 20, 20, 30 each for rounds 1 through 4 respectively. 600 µL of neutralized eluate was added to 10 mL of mid exponential phase HBV88 or HB1251+pecan134 as appropriate for 30 min before titrating an aliquot while the rest was gently pelleted and plated on 15 cm diameter dishes. Overnight growth was followed by scraping large plates for glycerol stocking and liquid culture for M13K07 superinfection and display. Induction conditions are described above in phage display and ELISA. Polyclonal ELISAs utilized aliquots of the saved superinfected supernatants while monoclonal ELISAs were derived from the titration plates.

## Claims

1. A cell comprising:
a first nucleic acid comprising a first promoter and an expression cassette encoding a polypeptide of interest, a biotin substrate site, and a display protein, the expression cassette having a termination codon between the polypeptide of interest and the display protein; and
a second nucleic acid comprising a second promoter and a gene encoding a suppressor tRNA that recognizes the termination codon in the first nucleic acid.

2. A method of generating biotinylated polypeptides of interest, the method comprising:
producing a cell comprising a nucleic acid comprising a promoter and an expression cassette encoding a polypeptide of interest, a biotin substrate site, and a display protein, and the expression cassette having a termination codon between the polypeptide of interest and the display protein;
incubating the cell under conditions sufficient for expression of the polypeptide of interest with the biotin substrate site; and
providing an agent capable of biotinylating the polypeptide of interest at the biotin substrate site.

3. The method of claim 2, wherein the polypeptide of interest is obtained from an antibody library.

4. The method of claim 2, wherein the polypeptide of interest is a single domain antibody.

5. The method of claim 2, wherein the agent capable of biotinylating the polypeptide of interest is a biotin ligase.

6. A method of generating biotinylated polypeptides of interest, the method comprising:
producing a cell comprising
a first nucleic acid comprising a first promoter and an expression cassette encoding a polypeptide of interest, a biotin substrate site, and a display protein, and the expression cassette having a termination codon between the polypeptide of interest and the display protein; and
a second nucleic acid comprising a second promoter and a gene encoding a suppressor tRNA that recognizes the termination codon in the first nucleic acid;
incubating the cell under conditions sufficient for protein expression from the first promoter in the first nucleic acid and the second promoter in the second nucleic acid, thereby the polypeptide of interest with the biotin substrate site and the display protein is produced; and
providing an agent capable of biotinylating the polypeptide of interest at the biotin substrate site, thereby producing biotinylated polypeptides of interest with the display protein.

7. A method of screening for a polypeptide of interest with affinity to a molecule of interest, the method comprising:
providing an array comprising biotin affinity agents;
producing biotinylated polypeptides comprising
producing a cell comprising a nucleic acid comprising a promoter and an expression cassette encoding a polypeptide of interest, a biotin substrate site, and a display protein, and the expression cassette having a termination codon between the polypeptide of interest and the display protein ;
incubating the cell under conditions sufficient for expression of the polypeptide of interest with the biotin substrate site; and providing an agent capable of biotinylating the polypeptide of interest at the biotin substrate site;
adding a cellular fraction comprising the biotinylated polypeptides to the array;
adding a molecule of interest to bind to the biotinylated polypeptides;
adding the cellular fraction comprising the biotinylated polypeptides; and
detecting the polypeptide of interest that exhibits binding affinity to the molecule of interest.

8. The method of claim 7, wherein the promoter is an inducible promoter.

9. The method of claim 7, wherein the polypeptide of interest is obtained from an antibody library, or wherein the polypeptide of interest is a single domain antibody.

10. The method of claim 7, wherein the expression cassette encodes a linker between the polypeptide of interest and the biotin substrate site.

11. The method of claim 7, wherein the agent capable of biotinylating the polypeptide of interest is a biotin ligase.

12. The method of claim 10, wherein the polypeptide of interest is obtained from an antibody library.

13. The method of claim 10, wherein the polypeptide of interest is a single domain antibody.

14. The method of claim 10, wherein the agent capable of biotinylating the polypeptide of interest is a biotin ligase.

## Patentansprüche

1. Zelle, umfassend:
eine erste Nukleinsäure umfassend einen ersten Promoter und eine Expressionskassette codierend ein Polypeptid von Interesse, eine Biotinsubstratstelle und ein Displayprotein, wobei die Expressionskassette ein Terminationscodon zwischen dem Polypeptid von Interesse und dem Displayprotein aufweist; und
eine zweite Nukleinsäure umfassend einen zweiten Promoter und ein Gen codierend eine Suppressor-tRNA, die das Terminationscodon in der ersten Nukleinsäure erkennt.

2. Verfahren zum Erzeugen biotinylierter Polypeptide von Interesse, wobei das Verfahren Folgendes umfasst:
Produzieren einer Zelle umfassend eine Nukleinsäure umfassend einen Promoter und eine Expressionskassette codierend ein Polypeptid von Interesse, eine Biotinsubstratstelle und ein Displayprotein und wobei die Expressionskassette ein Terminationscodon zwischen dem Polypeptid von Interesse und dem Displayprotein aufweist;
Inkubieren der Zelle unter Bedingungen, die zur Expression des Polypeptids von Interesse mit der Biotinsubstratstelle ausreichen; und
Bereitstellen eines Wirkstoffs, der fähig ist zum Biotinylieren des Polypeptids von Interesse an der Biotinsubstratstelle.

3. Verfahren nach Anspruch 2, worin das Polypeptid von Interesse aus einer Antikörperbank stammt.

4. Verfahren nach Anspruch 2, worin das Polypeptid von Interesse ein Einzeldomänen-Antikörper ist.

5. Verfahren nach Anspruch 2, worin der zum Biotinylieren des Polypeptids von Interesse fähige Wirkstoff eine Biotinligase ist.

6. Verfahren von Erzeugen biotinylierter Polypeptide von Interesse, wobei das Verfahren Folgendes umfasst:
Produzieren einer Zelle umfassend
eine erste Nukleinsäure umfassend einen ersten Promoter und eine Expressionskassette codierend ein Polypeptid von Interesse, eine Biotinsubstratstelle und ein Displayprotein und wobei die Expressionskassette ein Terminationscodon zwischen dem Polypeptid von Interesse und dem Displayprotein aufweist; und
eine zweite Nukleinsäure umfassend einen zweiten Promoter und ein Gen codierend eine Suppressor-tRNA, die das Terminationscodon in der ersten Nukleinsäure erkennt;
Inkubieren der Zelle unter Bedingungen, die zur Proteinexpression aus dem ersten Promoter in der ersten Nukleinsäure und dem zweiten Promoter in der zweiten Nukleinsäure ausreichen, wodurch das Polypeptid von Interesse mit der Biotinsubstratstelle und dem Displayprotein produziert wird; und
Bereitstellen eines Wirkstoffs, der fähig ist zum Biotinylieren des Polypeptids von Interesse an der Biotinsubstratstelle, wodurch biotinylierte Polypeptide von Interesse mit dem Displayprotein produziert werden.

7. Verfahren zum Screenen auf ein Polypeptid von Interesse mit Affinität zu einem Molekül von Interesse, wobei das Verfahren Folgendes umfasst:
Bereitstellen eines Arrays umfassend Biotinaffinitätswirkstoffe;
Produzieren biotinylierter Polypeptide umfassend
Produzieren einer Zelle umfassend eine Nukleinsäure umfassend einen Promoter und eine Expressionskassette codierend ein Polypeptid von Interesse, eine Biotinsubstratstelle und ein Displayprotein und wobei die Expressionskassette ein Terminationscodon zwischen dem Polypeptid von Interesse und dem Displayprotein aufweist;
Inkubieren der Zelle unter Bedingungen, die zur Expression des Polypeptids von Interesse mit der Biotinsubstratstelle ausreichen; und
Bereitstellen eines Wirkstoffs, der fähig ist zum Biotinylieren des Polypeptids von Interesse an der Biotinsubstratstelle,
Hinzufügen einer Zellfraktion umfassend die biotinylierten Polypeptide zum Array;
Hinzufügen eines Moleküls von Interesse zum Binden an die biotinylierten Polypeptide;
Hinzufügen der Zellfraktion umfassend die biotinylierten Polypeptide; und
Nachweisen des Polypeptids von Interesse, das Bindungsaffinität zum Molekül von Interesse aufweist.

8. Verfahren nach Anspruch 7, worin der Promoter ein induzierbarer Promoter ist.

9. Verfahren nach Anspruch 7, worin das Polypeptid von Interesse aus einer Antikörperbank stammt oder worin das Polypeptid von Interesse ein Einzeldomänen-Antikörper ist.

10. Verfahren nach Anspruch 7, worin die Expressionskassette einen Linker zwischen dem Polypeptid von Interesse und der Biotinsubstratstelle codiert.

11. Verfahren nach Anspruch 7, worin der zum Biotinylieren des Polypeptids von Interesse fähige Wirkstoff eine Biotinligase ist.

12. Verfahren nach Anspruch 10, worin das Polypeptid von Interesse aus einer Antikörperbank stammt.

13. Verfahren nach Anspruch 10, worin das Polypeptid von Interesse ein Einzeldomänen-Antikörper ist.

14. Verfahren nach Anspruch 10, worin der zum Biotinylieren des Polypeptids von Interesse fähige Wirkstoff eine Biotinligase ist.

## Revendications

1. Cellule comprenant:
un premier acide nucléique comprenant un premier promoteur et une cassette d'expression codant pour un polypeptide d'intérêt, un site de substrat pour la biotine, et une protéine d'affichage, la cassette d'expression ayant un codon de terminaison situé entre le polypeptide d'intérêt et la protéine d'affichage ; et
un deuxième acide nucléique comprenant un deuxième promoteur et un gène codant pour un ARNt suppresseur qui reconnaît le codon de terminaison dans le premier acide nucléique.

2. Procédé pour générer des polypeptides biotinylés d'intérêt, le procédé comprenant les étapes consistant à :
produire une cellule comprenant un acide nucléique comprenant un promoteur et une cassette d'expression codant pour un polypeptide d'intérêt, un site de substrat pour la biotine, et une protéine d'affichage, et la cassette d'expression ayant un codon de terminaison situé entre le polypeptide d'intérêt et la protéine d'affichage;
incuber la cellule sous des conditions suffisantes pour l'expression du polypeptide d'intérêt avec le site de substrat pour la biotine ; et
fournir un agent apte à biotinyler le polypeptide d'intérêt au site de substrat pour la biotine.

3. Procédé selon la revendication 2, dans lequel le polypeptide d'intérêt est issu d'une banque d'anticorps.

4. Procédé selon la revendication 2, dans lequel le peptide d'intérêt est un anticorps à domaine unique.

5. Procédé selon la revendication 2, dans lequel l'agent apte à biotinyler le polypeptide d'intérêt est une biotine-ligase.

6. Procédé pour générer des polypeptides biotinylés d'intérêt, le procédé comprenant les étapes consistant à :
produire une cellule comprenant
un premier acide nucléique comprenant un premier promoteur et une cassette d'expression codant pour un polypeptide d'intérêt, un site de substrat pour la biotine, et une protéine d'affichage, et la cassette d'expression ayant un codon de terminaison situé entre le polypeptide d'intérêt et la protéine d'affichage ; et
un deuxième acide nucléique comprenant un deuxième promoteur et un gène codant pour un tARN suppresseur qui reconnaît le codon de terminaison dans le premier acide nucléique ;
incuber la cellule sous des conditions suffisantes pour l'expression de la protéine issue du premier promoteur dans le premier acide nucléique et du deuxième promoteur dans le deuxième acide nucléique, si bien que le polypeptide d'intérêt avec le site de substrat pour la biotine et la protéine d'affichage est produit ; et fournir un agent apte à biotinyler le polypeptide d'intérêt au site de substrat pour la biotine, en produisant ainsi des polypeptides biotinylés d'intérêt avec la protéine d'affichage.

7. Procédé pour dépister un polypeptide d'intérêt qui a une affinité pour une molécule d'intérêt, le procédé comprenant les étapes consistant à :
fournir une matrice comprenant des agents d'affinité pour la biotine ; produire des polypeptides biotinylés comprenant les étapes consistant à :
produire une cellule comprenant un acide nucléique comprenant un promoteur et une cassette d'expression codant pour un polypeptide d'intérêt, un site de substrat pour la biotine et une protéine d'affichage, et la cassette d'expression ayant un codon de terminaison situé entre le polypeptide d'intérêt et la protéine d'affichage ;
incuber la cellule sous des conditions suffisantes pour l'expression du polypeptide d'intérêt avec le site de substrat pour la biotine ; et fournir un agent apte à biotinyler le polypeptide d'intérêt au site de substrat pour la biotine ; ajouter une fraction cellulaire comprenant les polypeptides biotinylés à la matrice ; ajouter une molécule d'intérêt pour l'apparier aux polypeptides biotinylés ; ajouter la fraction cellulaire comprenant les polypeptides biotinylés ; et détecter le polypeptide d'intérêt qui présente une affinité d'appariement à la molécule d'intérêt.

8. Procédé selon la revendication 7, dans lequel le promoteur est un promoteur inductible.

9. Procédé selon la revendication 7, dans lequel le polypeptide d'intérêt est issu d'une banque d'anticorps, ou dans lequel le polypeptide d'intérêt est un anticorps à domaine unique.

10. Procédé selon la revendication 7, dans lequel la cassette d'expression code pour un liant entre le polypeptide d'intérêt et le site de substrat pour la biotine.

11. Procédé selon la revendication 7, dans lequel l'agent apte à biotinyler le polypeptide d'intérêt est une biotine-ligase.

12. Procédé selon la revendication 10, dans lequel le polypeptide d'intérêt est issu d'une banque d'anticorps.

13. Procédé selon la revendication 10, dans lequel le polypeptide d'intérêt est un anticorps à domaine unique.

14. Procédé selon la revendication 10, dans lequel l'agent apte à biotinyler le polypeptide d'intérêt est une biotine-ligase.
